(19) Europäisches Patentamt
European Patent Office
Office européen des brevets

(11) **EP 2 806 271 B1**

(12) **EUROPÄISCHE PATENTSCHRIFT**

(45) Veröffentlichungstag und Bekanntmachung des Hinweises auf die Patenterteilung:
**26.04.2017 Patentblatt 2017/17**

(51) Int Cl.:
**G01N 33/22** *(2006.01)*   **G01N 7/00** *(2006.01)*

(21) Anmeldenummer: **14001767.4**

(22) Anmeldetag: **20.05.2014**

(54) **Verfahren und Messvorrichtung zur Bestimmung von physikalischen Gaseigenschaften**

Method and measuring device for determining physical gas properties

Procédé et dispositif de mesure pour la détermination de propriétés physiques de gaz

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.05.2013 EP 13002708**

(43) Veröffentlichungstag der Anmeldung:
**26.11.2014 Patentblatt 2014/48**

(73) Patentinhaber: **MEMS AG**
**5200 BRUGG (CH)**

(72) Erfinder:
• **Prêtre, Philippe**
**5405 Dättwil (CH)**

• **Kempe, Andreas**
**8049 Zürich (CH)**
• **Suter, Tobias**
**8802 Kilchberg (CH)**

(74) Vertreter: **Steiner, Peter**
**Patente + Marken**
**Untere Wolfackerstrasse 16**
**8280 Kreuzlingen (CH)**

(56) Entgegenhaltungen:
**EP-A1- 1 265 068     EP-A1- 2 015 056**
**EP-A1- 2 574 918     EP-A2- 0 591 639**

**EP 2 806 271 B1**

**Beschreibung**

Die Erfindung bezieht sich auf ein Verfahren und auf eine Messvorrichtung zur Bestimmung physikalischer Eigenschaften und brenntechnisch relevanter Grössen von Gasen und Gasgemischen. Unter physikalischen Gaseigenschaften werden insbesondere die Dichte, Wärmeleitfähigkeit, Wärmekapazität und Viskosität und die daraus korrelierbaren, brenntechnisch relevanten Grössen wie der Energieinhalt, Brennwert, Wobbe-Index, die Methanzahl und/oder der Luftbedarf des Gases oder Gasgemisches verstanden.

Bei Gasfeuerungsregelungen ist es wichtig, bei wechselnder Brenngasqualität die Brennerbelastung konstant zu halten. Der entsprechende Index zur Anzeige der Austauschbarkeit von Gasen ist der Wobbe-Index, gebildet aus dem Brennwert und der Wurzel aus dem Dichteverhältnis zwischen Luft und diesem Gas. Bei gleichem Wobbe-Index ergibt sich dann die gleiche Wärmebelastung eines Brenners.

Bei der Regelung von (Erd-)Gasmotoren ist zur Leistungs- bzw. Effizienzsteigerung die Kenntnis des Brennwerts bei wechselnden (Erd-)Gasqualitäten wichtig, zur Beurteilung des Zündverhaltens (Klopfen bzw. Zündaussetzer) wird hingegen bei Gas die zur Oktanzahl bei Benzin analoge Methanzahl herangezogen.

Für einen optimalen Verbrennungsprozess ist das richtige Mischverhältnis zwischen Brenngas und Luft wichtig, der sogenannte Luftbedarf. Bei zuwenig Luft bildet sich normalerweise Russ (Abgase), was vor allem bei Brennstoffzellen zu deren Zerstörung führen kann. Zuviel Luft bei der Verbrennung resultiert in einer kleineren Leistungsausbeute. Dabei hängt der optimale Wert von der jeweiligen Anwendung ab, ändert sich aber wieder bei wechselnder Gasqualität.

Aus der Literatur sind Korrelationsmethoden zur Berechnung brenntechnisch relevanter Größen bekannt, siehe zum Beispiel U. Wernekinck, "Gasmessung und Gasabrechung", Vulkan Verlag, 2009, ISBN 978-3-8027-5620-7. Dabei werden folgende Kombinationen von Messgrössen verwendet:

    A. Dielektrizitätskonstante, Schallgeschwindigkeit, $CO_2$-Gehalt

    B. Schallgeschwindigkeit bei 2 Drücken, $CO_2$-Gehalt

    C. Wärmeleitfähigkeit bei 2 Temperaturen, Schallgeschwindigkeit

    D. Wärmeleitfähigkeit, Wärmekapazität, dynamische Viskosität

    E. Wärmeleitfähigkeit, Infrarot Absorption (nicht dispersiv)

    F. Infrarot Absorption (dispersiv)

Kommerziell erhältliche Geräte, die für eichpflichtige Brennwertmessungen zugelassen sind, gibt es zurzeit nur wenige, z.B. das Gerät EMC500 von RMG-Honeywell (Typus D plus $CO_2$-Gehalt), oder das Gerät Gas-lab Q1 von Elster-Instromet (Typus E plus $CO_2$-Gehalt). Wegen des hohen Einstandspreises ist jedoch keines dieser Geräte für den Massenmarkt geeignet.

Integrierte CMOS-Hitzdrahtanemometer ermöglichen sowohl eine mikrothermische Wärmeleitfähigkeitsmessung als auch eine Massenflussmessung. Zu dieser Technologie wird auf D. Matter, B. Kramer, T. Kleiner, B. Sabbattini, T. Suter, "Mikroelektronischer Haushaltsgaszähler mit neuer Technologie", Technisches Messen 71, 3 (2004), S. 137-146 hingewiesen. Sie unterscheidet sich insbesondere von herkömmlichen, thermischen Massenflussmessern durch eine Messung direkt im Gasstrom und nicht von aussen an einer den Gasstrom umfassenden Metallkapillare.

Aus EP 2 015 056 A1 ist ein thermischer Durchflusssensor zur Bestimmung einer brenntechnisch relevanten Grösse bekannt, basierend auf einer Wärmeleitfähigkeitsmessung bei quasi bekanntem Massenfluss. Dazu wird eine kritische Düse benutzt, um den Massenfluss konstant zu halten, und es wird versucht, die Gasartenabhängigkeit der kritischen Düse mittels der Wärmeleitfähigkeit zu korrigieren. Die Information zur Korrelation brenntechnisch relevanter Grössen ist jedoch auf zwei quasi unabhängige Messwerte beschränkt, sodass keine Validierung der Messdaten möglich ist.

Aus WO 2004/036209 A1 ist ein Sensor zur Bestimmung brenntechnisch relevanter Grössen bekannt, bei dem der Massenfluss konstant gehalten wird und mittels einer thermischen Messung eine zur Wärmekapazität proportionale Grösse ermittelt wird. Da es sich nicht um einen mikrothermischen Sensor handelt, kann nicht auf die Wärmeleitfähigkeit geschlossen werden, womit die Bestimmung der Wärmekapazität und der daraus abgeleiteten, brenntechnisch relevanten Grössen nur bis auf einen Proportionalitätsfaktor möglich ist, was eine zusätzliche Kalibrierung mit bekannten Gaszusammensetzungen notwendig macht. Zudem entfällt die Information über die Wärmeleitfähigkeit und damit die Möglichkeit der Korrelation der Wärmeleitfähigkeit $\lambda$ mit einer der brenntechnisch relevanten Grössen. Im Weiteren ist die Genauigkeit dieser Methode limitiert durch die auftretenden Änderungen der nicht zugänglichen Wärmeleitfähigkeit $\lambda$.

Der Erfindung liegt daher die Aufgabe zu Grunde, ein Verfahren und eine Messvorrichtung zur Bestimmung physikalischer Eigenschaften von Gasen und Gasgemischen anzugeben, mit denen eine höhere Genauigkeit erzielt werden kann als mit den Sensoren aus den oben beschriebenen Patentdokumenten, wobei es möglich ist, die Messvorrichtung zu tieferen Kosten herzustellen als kommerziell erhältliche Geräte, die für eichpflichtige Brennwertmessungen zugelassen sind.

Die Aufgabe wird durch ein Verfahren nach Anspruch 1 und durch eine Messvorrichtung nach Anspruch 13 gelöst.

Die Idee der Erfindung ist die Bestimmung physikalischer Gaseigenschaften beruhend auf einer Druckabfallmessung eines vorgegebenen Volumens an Gas durch eine kritische Düse in Kombination mit einem mikrothermischen Sensor, mit dem sowohl Durchfluss wie auch Wärmeleitfähigkeit gemessen werden kann. Druckabfall- und Durchflussmessung können auf Konsistenz validiert werden, da der mikrothermische Sensor mit demselben Massenfluss beaufschlagt wird, der auch durch die kritische Düse fliesst.

Aus den drei Messwerten lassen sich weitere Grössen über Korrelationen ermitteln.

Druckabfallmessung eines vorgegebenen Volumens an Gas durch eine kritische Düse:

Der Massenfluss $\dot{m}$ durch eine kritische Düse ist gegeben durch

$$\dot{m} = C_d \cdot p \cdot A^* \cdot \psi_{\max} \cdot \sqrt{\frac{M}{T \cdot R_m}} \,, \qquad (1)$$

worin $C_d$ den "Discharge Coefficient", d.h. den Verlustfaktor einer realen gegenüber einer idealen kritischen Düse, $p$ den Vordruck, $A^*$ den Düsenquerschnitt, $T$ die Vortemperatur, $R_m$ die universelle Gaskonstante, $M$ das Molekulargewicht des Gases und $\psi_{max}$ den Maximalwert der Ausflussfunktion bezeichnet. Letztere ist eine Funktion des isentropen Koeffizienten $\gamma = c_p/c_V$ (Verhältnis von isobarer zu isochorer Wärmekapazität),

$$\psi = \sqrt{\gamma} \cdot \left( \frac{\gamma + 1}{2} \right)^{\frac{\gamma + 1}{2(1 - \gamma)}} . \qquad (2)$$

Lässt man aus einem bekanntem Volumen V das Gas von einem hohen Druck über die kritische Düse entspannen (z.B. von 9 auf 4 bar), ist der Druck in dem Volumen aufgrund des idealen Gasgesetzes wie folgt von der Zeit $t$ abhängig:

$$p(t) = m(t) \cdot \frac{R_m \cdot T}{M \cdot V} . \qquad (3)$$

Die Änderungsrate des Druckes ergibt sich somit zu

$$\frac{dp(t)}{dt} = \frac{dm(t)}{dt} \cdot \frac{R_m \cdot T}{M \cdot V} = \dot{m}(t) \cdot \frac{R_m \cdot T}{M \cdot V} \qquad (4)$$

und zusammen mit Gleichung (1) zu

$$\frac{dp(t)}{dt} = C_d \cdot p \cdot A^* \cdot \psi_{\max} \cdot \sqrt{\frac{M}{T \cdot R_m}} \cdot \frac{R_m \cdot T}{M \cdot V}$$

$$= C_d \cdot \frac{A^* \cdot \psi_{\max}}{V} \cdot \sqrt{\frac{R_m \cdot T}{M}} \cdot p(t) \qquad . \quad (5)$$

Misst man also den Verlauf des Druckes in Abhängigkeit der Zeit, kann man so die Zeitkonstante $\tau$ der durch Integration erhaltenen, zugehörigen Exponentialfunktion bestimmen:

$$1/\tau \; = \frac{C_d \cdot A^* \cdot \psi_{\max}}{V} \cdot \sqrt{\frac{R_m \cdot T}{M}} \; . \qquad (6)$$

Kennt man durch Messen zusätzlich die Temperatur $T$, kann man durch Weglassen aller nicht gasabhängigen Grössen einen Gaseigenschaftsfaktor

$$\Gamma^* := \; C_d \cdot \psi_{\max} \cdot \sqrt{\frac{1}{M}} \qquad (7)$$

definieren.

Lässt man umgekehrt das Gas von einem höheren Druck über die kritische Düse in ein bekanntes Volumen $V$ entspannen (z.B. von Umgebungsdruck nach Vakuum), lautet Gleichung (5') für die Druckzuname im Volumen $V$ wie folgt:

$$\begin{aligned}
\frac{dp(t)}{dt} &= C_d \cdot p_{Nozzle} \cdot A^* \cdot \psi_{\max} \cdot \sqrt{\frac{M}{T \cdot R_m}} \cdot \frac{R_m \cdot T}{M \cdot V} \\
&= C_d \cdot \frac{A^* \cdot \psi_{\max}}{V} \cdot \sqrt{\frac{R_m \cdot T}{M}} \cdot p_{Nozzle}
\end{aligned} \qquad , \quad (5')$$

wobei in diesem Fall der Druck vor der Düse, $p_{Nozzle}$, konstant ist, was zu einem mit der Zeit linearen Druckanstieg im Volumen $V$ führt mit

$$\frac{C_d \cdot A^* \cdot \psi_{\max}}{V} \cdot \sqrt{\frac{R_m \cdot T}{M}} \cdot p_{Nozzle} \qquad (6')$$

als Proportionalitätskonstante. Kennt man durch Messen zusätzlich die Temperatur $T$ und den Düsenvordruck $p_{Nozzle}$, kann man durch Weglassen aller nicht gasabhängigen Grössen wiederum den Gaseigenschaftsfaktor

$$\Gamma^* := \; C_d \cdot \psi_{\max} \cdot \sqrt{\frac{1}{M}} \qquad (7')$$

definieren.

Massenflussmessung mittels mikrothermischen Sensors:

Zur Beschreibung der mikrothermischen Massenflussmessung wird von der das mikrothermische System beschreibenden, eindimensionalen Wärmeleitungsgleichung ausgegangen (Kerson Huang: Statistical Mechanics, 2. Auflage, John Wiley & Sons, New York 1987, ISBN 0-471-85913-3):

$$\frac{c_p}{\lambda} \cdot \rho \, v_x \cdot \frac{d}{dx} T = \nabla^2 T + \frac{1}{\lambda} \Theta \, , \qquad (8)$$

wobei

$v_x$ die Komponente der mittleren Fliessgeschwindigkeit (Geschwindigkeitsvektor) $\vec{v}$ in x-Richtung, d. h. entlang des Gasstromes,

$T$ die Temperatur,

$\frac{d}{dx} T$ der Temperaturgradient,

$c_p$ die Wärmekapazität des Gases bei konstantem Druck,

$\rho$ die Dichte,

$\lambda$ die Wärmeleitfähigkeit des Gases,

$\nabla^2 T$ der Laplace-Operator, angewandt auf die Temperatur $T$, wobei

$$\nabla^2 = \left(\frac{d}{d_x}\right)^2 + \left(\frac{d}{dy}\right)^2 + \left(\frac{d}{dz}\right)^2 \, ,$$

bedeuten. Da das Gas (Gasstrom) nur in x-Richtung fliesst, werden die Komponenten $v_y$ bzw. $v_z$ in y-Richtung bzw. in z-Richtung der mittleren Fliessgeschwindigkeit $\vec{v}$ zu Null angenommen. $\Theta$ mit der Einheit Watt/m$^3$ beschreibt den Quellterm des Heizelements. Der Quellterm rührt bei der mikrothermischen Methode vom Heizdraht eines miniaturisierten, integrierten Hitzdrahtanemometers her, der Wärmeenergie in das System speist. Die Lösung der Gleichung (8), die die Temperaturverteilung im mikrothermischen System beschreibt, erlaubt es, durch das Messen dieser Temperaturverteilung den Faktor $S$,

$$S := \frac{c_p}{\lambda} \cdot \rho \cdot v_x \ = \ \frac{c_p}{\lambda} \cdot \frac{\dot{m}}{A} \, , \qquad (9)$$

zu bestimmen, wobei $A$ den Querschnitt des Flusskanals über dem mikrothermischen Sensor bezeichnet. In Kombination mit der kritischen Düse, d.h. durch Anordnen des mikrothermischen Sensors nach der kritischen Düse, ist der Massenfluss aber durch Gleichung (1) gegeben, weswegen

$$\frac{c_p}{\lambda} \cdot \rho \cdot v_x \ = \ \frac{c_p}{\lambda} \cdot C_d \cdot p \cdot \frac{A^*}{A} \cdot \psi_{max} \cdot \sqrt{\frac{M}{T \cdot R_m}} \, . \qquad (10)$$

Mit dem Messen von Druck $p$ und Temperatur $T$ und wiederum durch Weglassen aller gasunabhängigen Grössen erhält man einen zweiten Gaseigenschaftsfaktor

$$\Gamma \ = \ \frac{c_p}{\lambda} \cdot C_d \cdot \psi_{max} \cdot \sqrt{M} \, . \qquad (11)$$

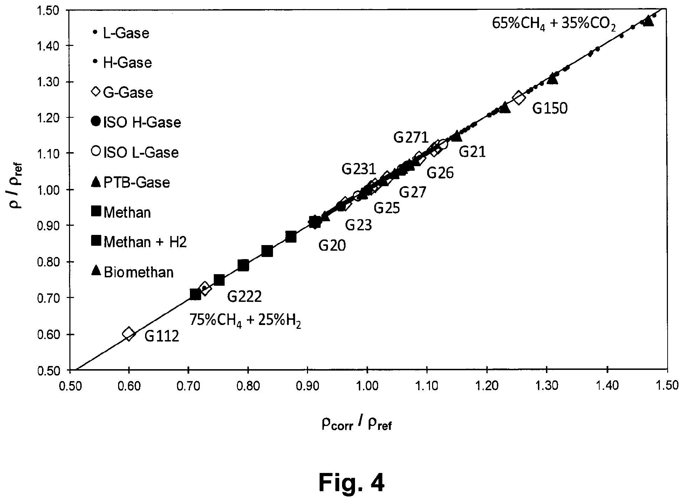

Das Weglassen aller gasunabhängigen Grössen in Gleichung (7) und (11) geschieht implizit, wenn man $\Gamma$ und $\Gamma^*$ ins Verhältnis zu $\Gamma$ und $\Gamma^*$ eines bekannten (Kalibrier-)Gases setzt. Siehe auch Fig. 4.

Messung der Wärmeleitfähigkeit mittels mikrothermischen Sensors:

Man beachte, dass die Wärmeleitfähigkeit $\lambda$ wegen des Quellterms $\Theta$ zusätzlich separat auf die Lösung der Gleichung (8) einwirkt. Umgekehrt kann die Wärmeleitfähigkeit bestimmt werden, wenn der mikrothermische Sensor ohne beaufschlagten Massenfluss ($v_x = 0$ bzw. $\dot{m} = 0$) gemessen wird. Die zugehörige Differentialgleichung für die Temperaturverteilung lautet dann einfach

$$\nabla^2 T = -\frac{1}{\lambda}\Theta \ .\qquad (12)$$

Validierung der Gaseigenschaftsfaktoren $\Gamma$ bzw. $\Gamma^*$:
Das Verhältnis der beiden Gaseigenschaftsfaktoren $\Gamma$ und $\Gamma^*$ ergibt

$$\Gamma/\Gamma^* = \frac{c_p}{\lambda}\cdot M \propto \frac{c_p}{\lambda}\cdot\rho_{norm} \ ,\qquad (13)$$

da das Molekulargewicht wegen des für die meisten Gase praktisch identischen Molvolumens proportional zur Normdichte (Dichte bei Normbedingungen 1013.25 mbar und 273.15 K) ist. Damit kann aus dem mittels mikrothermischen Sensors gemessenen Faktor $S$ in Gleichung (9) die Fliessgeschwindigkeit $v_x$ und zusammen mit dem Flusskanalquerschnitt $A$ der Normvolumenfluss $\phi_{norm} = v_x \cdot A$ extrahiert werden. Integration dieses Volumenflusses über die Zeit, d.h. im Zeitintervall $t_2$-$t_1$, sollte dann mit dem aus den entsprechenden Druck- und Temperaturwerten berechneten, abgeflossenen Gasvolumen übereinstimmen:

$$\int_{t_1}^{t_2}\phi_{norm}(t)\,dt \ \overset{!}{=}\ \frac{(p(t_2)-p(t_1))}{1013.25\ mbar}\cdot\frac{273.15\ K}{T}\cdot V \ .\ (14)$$

Stimmen die zwei Werte nicht überein, kann je nachdem, welche Grösse weniger genau gemessen werden kann, der Normvolumenfluss oder das Drucksignal soweit korrigiert werden, bis Gleichung (14) erfüllt ist. Im Fall einer Normvolumenflusskorrektur für $v_x = \phi_{norm} / A$ erfährt die rechte Seite von Gleichung (13) über den gemessenem Faktor $S$ in Gleichung (9) ebenfalls eine Korrektur und damit auch der Gaseigenschaftsfaktor $\Gamma$ wiederum über Gleichung (13). Im Fall einer Drucksignalkorrektur folgt eine korrigierte Zeitkonstante $\tau$ in Gleichung (6) bzw. eine korrigierte Proportionalitätskonstante in Gleichung (6') und damit eine Korrektur des Gaseigenschaftsfaktor $\Gamma^*$ in Gleichung (7) bzw. (7'). Auf diese Weise sind $\Gamma$ und $\Gamma^*$ konsistent bestimmt worden, denn der Massenfluss durch die Düse ist derselbe wie der Massenfluss, mit dem der mikrothermische Sensor beaufschlagt wird.

Korrelation brenntechnisch relevanter Grössen:

Mit der Messungen der Gaseigenschaftsfaktoren $\Gamma$ und $\Gamma^*$ sowie der Wärmeleitfähigkeit $\lambda$ stehen drei unabhängige Messgrössen zur Verfügung, mit denen nun brenntechnisch relevante Grössen $Q$ mittels einer Funktion $f_{corr}$ korreliert werden können:

$$Q_{corr} = f_{corr}(\Gamma,\Gamma^*,\lambda) \ .\qquad (15)$$

Beispielsweise ergibt sich für die Korrelation des in Fig. 4 gezeigten Dichteverhältnisses $\rho_{corr} / \rho_{ref}$ bei 0°C und 1013.25 mbar folgende Korrelationsfunktion:

$$\rho_{corr}/\rho_{ref} = f_{corr}(\Gamma,\Gamma^*,\lambda) = \Gamma^r\cdot\Gamma^{*s}\cdot\lambda^t\qquad (16)$$

mit Exponenten $r$ = -0.2, s = -1.8 und $t$ = -0.2 und einem typischen H-Erdgas als Referenz.

Verfahren und Messvorrichtung gemäss vorliegender Erfindung

In dem Verfahren zur Bestimmung physikalischer Eigenschaften und/oder brenntechnisch relevanter Grössen eines Gases und Gasgemisches gemäss vorliegender Erfindung:

-   fliesst das Gas oder Gasgemisch aus einem Gasreservoir durch eine kritische Düse und über einen mikrothermischen Sensor, wobei die kritische Düse und der mikrothermischen Sensor mit demselben Massenfluss beaufschlagt werden;

- wobei der Druckabfall im Gasreservoir als Funktion der Zeit gemessen wird;

- aus den Messwerten des Druckabfalls ein erster, von einer ersten Gruppe von physikalischen Eigenschaften des Gases oder Gasgemisches abhängiger Gaseigenschaftsfaktor $\Gamma^*$ bestimmt wird, wobei der erste Gaseigenschaftsfaktor zum Beispiel aus einer Zeitkonstante des Druckabfalls abgeleitet wird;

- aus dem Durchflusssignal des mikrothermischen Sensors ein zweiter, von einer zweiten Gruppe von physikalischen Eigenschaften des Gases oder Gasgemisches abhängiger Gaseigenschaftsfaktor $\Gamma$ bestimmt wird, wobei der zweite Gaseigenschaftsfaktor zum Beispiel die Wärmekapazität $c_p$ des Gases oder Gasgemisches enthält oder von derselben abhängig ist;

- mit Hilfe des mikrothermischen Sensors die Wärmeleitfähigkeit $\lambda$ des Gases oder Gasgemisches bestimmt wird; und

- aus dem ersten und/oder zweiten Gaseigenschaftsfaktor $\Gamma^*$, r und der Wärmeleitfähigkeit $\lambda$ mittels Korrelation eine gesuchte physikalische Eigenschaft oder brenntechnisch relevante Grösse ermittelt wird.

Vorteilhafterweise wird von einem exponentiellen Abfall des gemessenen Druckes ausgegangen und der erste Gaseigenschaftsfaktor $\Gamma^*$ aus der Zeitkonstante des Druckabfalls abgeleitet, wobei der erste Gaseigenschaftsfaktor gebildet wird, indem zum Beispiel zusätzlich die Temperatur $T$ gemessen und alle nicht gasabhängigen Grössen weggelassen werden.

Der zweite Gaseigenschaftsfaktor ($\Gamma$) enthält typisch den Quotienten Wärmekapazität $c_p$ geteilt durch Wärmeleitfähigkeit $\lambda$ des Gases oder Gasgemisches oder ist von demselben abhängig, wobei der zweite Gaseigenschaftsfaktor gebildet wird, indem zum Beispiel zusätzlich die Temperatur $T$ gemessen und alle nicht gasabhängigen Grössen weggelassen werden.

In einer vorteilhaften Ausführungsform des Verfahrens werden die Gaseigenschaftsfaktoren $\Gamma^*$, $\Gamma$ durch Vergleich der Werte für das Gesamtvolumen des ausgeströmten Gases oder Gasgemisches validiert, indem Druck und Temperatur im Gasreservoir am Anfang und am Schluss der Druckabfallmessung gemessen und das ausgeflossene Normvolumen bei bekanntem Volumen des Gasreservoirs bestimmt werden, der mittels des mikrothermischen Sensors gemessene Normdurchfluss über das Zeitintervall zwischen Anfang und Schluss der Druckabfallmessung aufsummiert wird, das ausgeflossene Normvolumen mit dem aufsummierten Normdurchfluss verglichen wird, und bei Ungleichheit entweder der erste und/oder zweite Gaseigenschaftsfaktor korrigiert wird, zum Beispiel durch Korrektur des Drucksignals oder des Normvolumenflusswertes des mikrothermischen Sensors.

Die oben stehend beschriebene Ausführungsform des Verfahrens kann zur Kalibrierung des Durchflusssignals des mikrothermischen Sensors benutzt werden, indem das Durchflusssignal des mikrothermischen Sensors für ein bestimmtes Kalibriergas oder -Gasgemisch kalibriert wird, das Verhältnis $\Gamma / \Gamma^*$ des zweiten, aus dem Durchflusssignal des mikrothermischen Sensors bestimmten Gaseigenschaftsfaktors zum ersten Gaseigenschaftsfaktor für ein unbekanntes Gas oder Gasgemisch bestimmt wird, und die Normvoluminawerte aus der Messung des Druckabfalls und des aufsummierten Normdurchflusses des mikrothermische Sensors verglichen und dazu verwendet werden, das Verhältnis des zweiten zum ersten Gaseigenschaftsfaktor zu korrigieren und den Wert für den zweiten Gaseigenschaftsfaktor $\Gamma$ anzupassen.

In einer weiteren vorteilhaften Ausführungsform des Verfahrens zur Bestimmung physikalischer Eigenschaften und/oder brenntechnisch relevanter Grössen eines Gases oder Gasgemisches:

- fliesst das Gas oder Gasgemisch unter Druck durch eine kritische Düse und über einen mikrothermischen Sensor in ein Gasreservoir, wobei die kritische Düse und der mikrothermischen Sensor mit demselben Massenfluss beaufschlagt werden;

- wobei die Druckzunahme im Gasreservoir als Funktion der Zeit gemessen wird;

- aus den Messwerten der Druckzunahme ein erster, von einer ersten Gruppe von physikalischen Eigenschaften des Gases oder Gasgemisches abhängiger Gaseigenschaftsfaktor $\Gamma^*$ bestimmt wird;

- aus dem Durchflusssignal des mikrothermischen Sensors ein zweiter, von einer zweiten Gruppe von physikalischen Eigenschaften des Gases oder Gasgemisches abhängiger Gaseigenschaftsfaktor $\Gamma$ bestimmt wird, wobei der zweite Gaseigenschaftsfaktor zum Beispiel die Wärmekapazität $c_p$ des Gases oder Gasgemisches enthält oder von derselben abhängig ist;

- mit Hilfe des mikrothermischen Sensors die Wärmeleitfähigkeit λ des Gases oder Gasgemisches bestimmt wird; und

- aus dem ersten und/oder zweiten Gaseigenschaftsfaktor Γ*, Γ und der Wärmeleitfähigkeit λ mittels Korrelation eine gesuchte physikalische Eigenschaft oder brenntechnisch relevante Grösse ermittelt wird.

Vorteilhafterweise wird von einer linearen Zunahme des gemessenen Druckes ausgegangen und der erste Gaseigenschaftsfaktor Γ* aus der Proportionalitätskonstante der Druckzunahme abgeleitet, wobei der erste Gaseigenschaftsfaktor gebildet wird, indem zum Beispiel zusätzlich die Temperatur $T$ und der Düsenvordruck $p_{Nozzle}$ gemessen und alle nicht gasabhängigen Grössen weggelassen werden.

Der zweite Gaseigenschaftsfaktor Γ enthält typisch den Quotienten Wärmekapazität $c_p$ geteilt durch Wärmeleitfähigkeit λ des Gases oder Gasgemisches oder ist von demselben abhängig, wobei der zweite Gaseigenschaftsfaktor gebildet wird, indem zum Beispiel zusätzlich die Temperatur $T$ gemessen und alle nicht gasabhängigen Grössen weggelassen werden.

In einer weiteren vorteilhaften Ausführungsform des Verfahrens werden die Gaseigenschaftsfaktoren Γ*, r durch Vergleich der Werte für das Gesamtvolumen des ins Gasreservoir geflossenen Gases oder Gasgemisches validiert, indem Druck und Temperatur im Gasreservoir am Anfang und am Schluss der Druckzunahmemessung gemessen werden, und das ins Gasreservoir geflossene Normvolumen bei bekanntem Volumen des Gasreservoirs bestimmt wird, der mittels des mikrothermischen Sensors gemessene Normdurchfluss über das Zeitintervall zwischen Anfang und Schluss der Druckzunahmemessung aufsummiert wird, das ins Gasreservoir geflossene Normvolumen mit dem aufsummierten Normdurchfluss verglichen wird, und bei Ungleichheit entweder der erste und/oder zweite Gaseigenschaftsfaktor korrigiert wird, zum Beispiel durch Korrektur des Drucksignals oder des Normvolumenflusswertes des mikrothermischen Sensors.

Die oben stehend beschriebene Ausführungsform des Verfahrens kann zur Kalibrierung des Durchflusssignals des mikrothermischen Sensors benutzt werden, indem das Durchflusssignal des mikrothermischen Sensors für ein bestimmtes Kalibriergas oder -Gasgemisch kalibriert wird, das Verhältnis Γ / Γ* des zweiten, aus dem Durchflusssignal des mikrothermischen Sensors bestimmten Gaseigenschaftsfaktors zum ersten Gaseigenschaftsfaktor für ein unbekanntes Gas oder Gasgemisch bestimmt wird, und die Normvoluminawerte aus der Messung der Druckzunahme und des aufsummierten Normdurchflusses des mikrothermische Sensors verglichen und dazu verwendet werden, das Verhältnis des zweiten zum ersten Gaseigenschaftsfaktor zu korrigieren und den Wert für den zweiten Gaseigenschaftsfaktor Γ anzupassen.

Die gesuchte physikalische Eigenschaft kann beispielsweise die Dichte oder die Wärmeleitfähigkeit oder die Wärmekapazität oder die Viskosität des Gases oder Gasgemisches sein, und die brenntechnisch relevante Grösse kann beispielsweise der Energieinhalt oder der Brennwert oder der Wobbe-Index oder die Methanzahl oder der Luftbedarf des Gases oder Gasgemisches sein.

Die gesuchte physikalische Eigenschaft oder brenntechnisch relevante Grösse Q wird vorteilhafterweise mittels einer Korrelationsfunktion $Q = f_{corr}(Γ, Γ^*, λ) = const \cdot Γ^r \cdot Γ^{*s} \cdot λ^t$ ermittelt, wobei $r,$ s und $t$ Exponenten sind und *const* eine Konstante ist.

Der Druck im Gasreservoir zu Beginn der Druckabfallmessung ist typisch grösser als der kritische Druck $p_{krit}$ der kritischen Düse und der Aussendruck nach der kritischen Düse kleiner als die Hälfte des kritischen Druckes, oder der Druck im Gasreservoir zu Beginn der Druckzunahmemessung ist typisch kleiner als die Hälfte des kritischen Druckes $p_{krit}$ der kritischen Düse und der Druck vor der kritischen Düse grösser ist als der kritische Druck.

Unabhängig von der Ausführungsform und -variante ist das Gasreservoir während der Messung typisch abgetrennt von der Gasversorgung. Das Volumen des Gasreservoirs wird vorteilhafterweise so gewählt, dass der Druck im Gasreservoir bis zum Ende der Messung deutlich ab- oder zunimmt, beispielsweise um mindestens einen Zehntel oder Fünftel des ursprünglichen Druckes.

Die Messvorrichtung zur Bestimmung physikalischer Eigenschaften und/oder brenntechnisch relevanter Grössen eines Gases oder Gasgemisches gemäss vorliegender Erfindung enthält eine Auswerteeinheit, die zur Ausführung eines Verfahrens gemäss einer der oben stehend beschriebenen Ausführungsformen und -varianten eingerichtet ist, sowie ein Gasreservoir, das mit einem Drucksensor versehen ist, eine kritische Düse und einen mikrothermischen Sensor zur Messung des Durchflusses und der Wärmeleitfähigkeit, wobei das Gasreservoir für die Messung mit der kritischen Düse und dem mikrothermischen Sensor verbunden ist.

Weiter umfasst die Erfindung auch die Verwendung eines Gasreservoirs und einer kritischen Düse zur Bestimmung physikalischer Eigenschaften und/oder brenntechnisch relevanter Grössen eines Gases oder Gasgemisches, wobei das Gas oder Gasgemisch unter Druck aus dem Gasreservoir durch die kritische Düse fliesst, wobei der Druckabfall im Gasreservoir als Funktion der Zeit gemessen wird, aus den Messwerten des Druckabfalls ein von physikalischen Eigenschaften des Gases oder Gasgemisches abhängiger Gaseigenschaftsfaktor Γ* bestimmt wird, der zum Beispiel aus einer Zeitkonstante des Druckabfalls abgeleitet wird, und aus dem Gaseigenschaftsfaktor Γ* mittels Korrelation eine gesuchte physikalische Eigenschaft oder brenntechnisch relevante Grösse ermittelt wird.

In einer anderen vorteilhaften Ausführungsform wird ein Unterdruck im Gasreservoir erzeugt, und das Gas oder Gasgemisch fliesst unter Druck durch die kritische Düse in das Gasreservoir, wobei die Druckzunahme im Gasreservoir als Funktion der Zeit gemessen und aus den Messwerten der Druckzunahme ein von physikalischen Eigenschaften des Gases oder Gasgemisches abhängiger Gaseigenschaftsfaktor Γ* bestimmt wird, aus dem mittels Korrelation eine gesuchte physikalische Eigenschaft oder brenntechnisch relevante Grösse ermittelt wird.

Die oben beschriebene Verwendung eines Gasreservoirs und einer kritischen Düse zur Bestimmung physikalischer Eigenschaften und/oder brenntechnisch relevanter Grössen eines Gases oder Gasgemisches beziehungsweise das entsprechende Verfahren, in dem ein Gasreservoir und eine kritischen Düse zur Bestimmung physikalischer Eigenschaften und/oder brenntechnisch relevanter Grössen eines Gases oder Gasgemisches verwendet werden, kann auch als eigenständige Erfindung betrachtet werden, die zusätzlich eine Messvorrichtung mit Auswerteeinheit, Gasreservoir und kritischer Düse umfassen kann, wobei die Auswerteeinheit für die Verwendung des Gasreservoirs und der kritischen Düse zur Bestimmung physikalischer Eigenschaften und/oder brenntechnisch relevanter Grössen eines Gases oder Gasgemisches beziehungsweise zur Ausführung des entsprechenden Verfahrens eingerichtet ist.

Zusätzlich umfasst die Erfindung die Verwendung eines Gasreservoirs und eines für ein bestimmtes Kalibriergas oder -Gasgemisch kalibrierten mikrothermischen Sensors zur Bestimmung physikalischer Eigenschaften und/oder brenntechnisch relevanter Grössen eines Gases oder Gasgemisches, wobei das Gas oder Gasgemisch unter Druck aus dem Gasreservoir über den mikrothermischen Sensor fliesst, wobei der mit dem für ein bestimmtes Kalibriergas oder -Gasgemisch kalibrierten mikrothermischen Sensor bestimmte Volumenfluss $v_x \cdot A$ aufsummiert und mit dem aus dem Gasreservoir ausgeflossenen Gasvolumen verglichen wird, aus dem Vergleich der beiden Volumina ein von physikalischen Eigenschaften des Gases oder Gasgemisches abhängiger Gaseigenschaftsfaktor $S / v'_x$ bestimmt wird, in dem $v'_x$ die aus dem ausgeflossenen Gasvolumen bestimmte Fliessgeschwindigkeit bezeichnet, und aus dem Gaseigenschaftsfaktor, der zum Beispiel durch $S / v'_x = c_p \cdot \rho / \lambda$ gegeben sein kann (siehe Gleichung (9)), mittels Korrelation eine gesuchte physikalische Eigenschaft oder brenntechnisch relevante Grösse ermittelt wird.

In einer anderen vorteilhaften Ausführungsform wird ein Unterdruck im Gasreservoir erzeugt, und das Gas oder Gasgemisch fliesst unter Druck über den mikrothermischen Sensor in das Gasreservoir, wobei der mit dem für ein bestimmtes Kalibriergas oder -Gasgemisch kalibrierten mikrothermischen Sensor bestimmte Volumenfluss $v_x \cdot A$ aufsummiert und mit dem in das Gasreservoir geflossenen Gasvolumen verglichen wird, aus dem Vergleich der beiden Volumina ein von physikalischen Eigenschaften des Gases oder Gasgemisches abhängiger Gaseigenschaftsfaktor $S / v'_x$ bestimmt wird, und aus dem Gaseigenschaftsfaktor, der zum Beispiel durch $S / v'_x = c_p \cdot \rho / \lambda$ gegeben sein kann (siehe Gleichung (9)), mittels Korrelation eine gesuchte physikalische Eigenschaft oder brenntechnisch relevante Grösse ermittelt wird.

In einer weiteren vorteilhaften Ausführungsvariante wird der Gasfluss durch Verschieben eines Kolbens erzeugt.

Die oben beschriebene Verwendung eines Gasreservoirs und eines für ein bestimmtes Kalibriergas oder -Gasgemisch kalibrierten, mikrothermischen Sensors zur Bestimmung physikalischer Eigenschaften und/oder brenntechnisch relevanter Grössen eines Gases oder Gasgemisches beziehungsweise das entsprechende Verfahren, in dem ein Gasreservoir und ein für ein bestimmtes Kalibriergas oder -Gasgemisch kalibrierter, mikrothermischer Sensor zur Bestimmung physikalischer Eigenschaften und/oder brenntechnisch relevanter Grössen eines Gases oder Gasgemisches verwendet werden, kann auch als eigenständige Erfindung betrachtet werden, die zusätzlich eine Messvorrichtung mit Auswerteeinheit, Gasreservoir und mikrothermischem Sensor umfassen kann, wobei die Auswerteeinheit für die Verwendung des Gasreservoirs und des mikrothermischen Sensors zur Bestimmung physikalischer Eigenschaften und/oder brenntechnisch relevanter Grössen eines Gases oder Gasgemisches beziehungsweise zur Ausführung des entsprechenden Verfahrens eingerichtet ist.

Das Verfahren und die Messvorrichtung zur Bestimmung physikalischer Eigenschaften und/oder brenntechnisch relevanter Grössen eines Gases oder Gasgemisches gemäss vorliegender Erfindung haben den Vorteil, dass für die Korrelation brenntechnisch relevanter Grössen drei unabhängige Messwerte zur Verfügung stehen. Dies erlaubt einerseits eine vergleichsweise hohe Genauigkeit bei der Bestimmung brenntechnisch relevanter Grössen, die sonst nur mit wesentlich teureren Geräten erzielt werden kann, und ermöglicht andererseits, die Messungen zu validieren und mögliche Abweichungen zu korrigieren.

Weitere Vorteile sind aus der nachstehenden Beschreibung ersichtlich.

Die Erfindung wird im Folgenden anhand der Zeichnungen näher erläutert. Es zeigen:

Fig. 1a     ein Ausführungsbeispiel des schematischen Aufbaus einer Messvorrichtung gemäss vorliegender Erfindung (Hochdruckvariante),

Fig. 1b     eine Ausführungsvariante zu dem in Fig. 1a gezeigten Ausführungsbeispiel,

Fig. 2     ein zweites Ausführungsbeispiele des schematischen Aufbaus einer Messvorrichtung gemäss vorliegender Erfindung (Niederdruckvariante),

Fig. 3     ein Ausführungsbeispiel eines mikrothermischen Sensors zur Verwendung in einer Messvorrichtung gemäss vorliegender Erfindung, und

Fig. 4     eine grafische Darstellung des direkt gemessenen Dichteverhältnisses (y-Achse) in Abhängigkeit des korrelierten Dichteverhältnisses (x-Achse) für verschieden Gasgruppen bei Normbedingungen (0°C, 1013.25 mbar).

Fig. 5a     ein Ausführungsbeispiel des schematischen Aufbaus einer Messvorrichtung gemäss einer zweiten Ausführungsform der Erfindung (Hochdruckvariante),

Fig. 5b     eine Ausführungsvariante zu dem in Fig. 5a gezeigten Ausführungsbeispiel,

Fig. 6     ein zweites Ausführungsbeispiele des schematischen Aufbaus einer Messvorrichtung gemäss der zweiten Ausführungsform der Erfindung (Niederdruckvariante),

Fig. 7     eine grafische Darstellung des direkt gemessenen Methananteils (y-Achse) in Abhängigkeit des korrelierten Methananteils (x-Achse) für ein binäres Rohbiogas (Methan und Kohlendioxid).

Fig. 8a     ein Ausführungsbeispiel des schematischen Aufbaus einer Messvorrichtung gemäss einer dritten Ausführungsform der Erfindung mit Gasreservoir und mikrothermischem Sensor (Hochdruckvariante),

Fig. 8b     eine Ausführungsvariante zu dem in Fig. 8a gezeigten Ausführungsbeispiel,

Fig. 9     ein zweites Ausführungsbeispiele des schematischen Aufbaus einer Messvorrichtung gemäss der dritten Ausführungsform der Erfindung mit Gasreservoir und mikrothermischem Sensor (Niederdruckvariante),

Fig. 10     eine grafische Darstellung der Klasseneinteilung von Erdgasgemischen anhand der Wärmediffusivität (y-Achse) bei gleichzeitiger Kenntnis der Wärmeleitfähigkeit $\lambda$ (x-Achse).

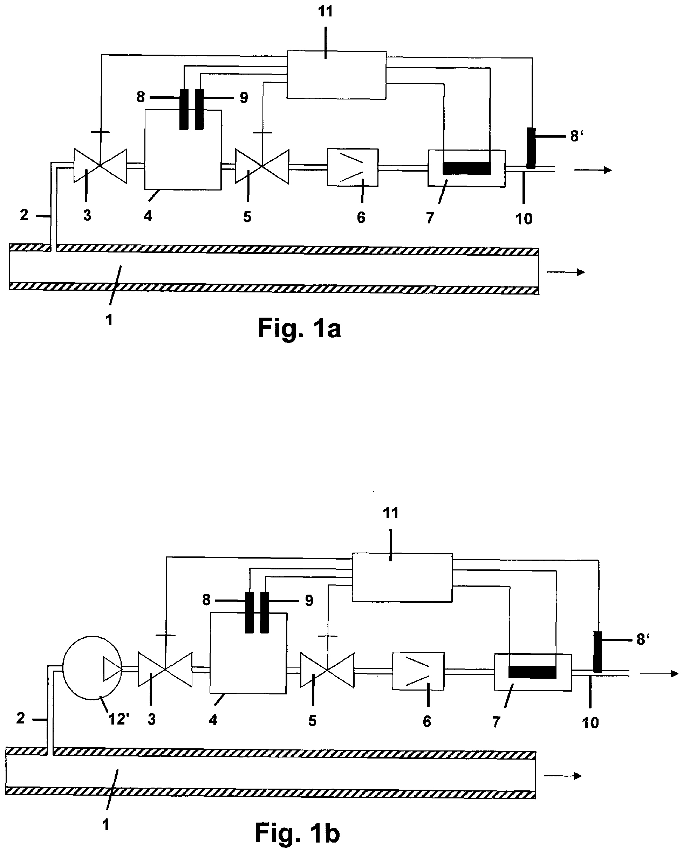

Fig. 1a zeigt ein Ausführungsbeispiel des schematischen Aufbaus einer Messvorrichtung gemäss vorliegender Erfindung im Fall, in dem die Gashauptleitung 1 einen Druck aufweist, der höher als der kritische Druck für die kritische Düse 6 der Messvorrichtung ist (Hochdruckvariante). Im Ausführungsbeispiel umfasst die Messvorrichtung zusätzlich zur kritischen Düse 6 eine Auswerteeinheit 11, die zur Ausführung eines Verfahrens gemäss vorliegender Erfindung eingerichtet ist, ein Gasreservoir 4, das mit einem Drucksensor 8 versehen ist, und einen mikrothermischen Sensor 7 zur Messung des Durchflusses und der Wärmeleitfähigkeit, wobei das Gasreservoir 4 für die Messung mit der kritischen Düse 6 und dem mikrothermischen Sensor 7 verbunden ist.

Bei Bedarf kann die Messvorrichtung zusätzlich eine oder mehrere der folgenden Komponenten enthalten: eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der am Auslass 10 angeordnet sein kann, einen Temperatursensor 9, der im Gasreservoir angeordnet ist, und einen Verdichter 12', der einlassseitig des Gasreservoirs 4 angeordnet sein kann, um den Druck im Gasreservoir zu erhöhen.

Ein Ausführungsbeispiel des Verfahrens zur Bestimmung physikalischer Eigenschaften und/oder brenntechnisch relevanter Grössen eines Gases und Gasgemisches gemäss vorliegender Erfindung wird im Folgenden anhand von Fig. 1a beschrieben. In dem Verfahren fliesst das Gas oder Gasgemisch aus dem Gasreservoir 4 durch die kritische Düse 6 und über den mikrothermischen Sensor 7, wobei die kritische Düse und der mikrothermischen Sensor mit demselben Massenfluss beaufschlagt werden. Der Druckabfall im Gasreservoir 4 wird als Funktion der Zeit gemessen und aus den Messwerten des Druckabfalls ein erster, von einer ersten Gruppe von physikalischen Eigenschaften des Gases oder Gasgemisches abhängiger Gaseigenschaftsfaktor $\Gamma^*$ bestimmt, wobei der erste Gaseigenschaftsfaktor zum Beispiel aus einer Zeitkonstante des Druckabfalls abgeleitet wird. Aus dem Durchflusssignal des mikrothermischen Sensors 7 wird ein zweiter, von einer zweiten Gruppe von physikalischen Eigenschaften des Gases oder Gasgemisches abhängiger Gaseigenschaftsfaktor $\Gamma$ bestimmt, wobei der zweite Gaseigenschaftsfaktor zum Beispiel die Wärmekapazität $c_p$ des Gases oder Gasgemisches enthält oder von derselben abhängig ist. Weiter wird mit Hilfe des mikrothermischen Sensors 7 die Wärmeleitfähigkeit λ des Gases oder Gasgemisches bestimmt und aus dem ersten und/oder zweiten Gaseigenschaftsfaktor Γ*, Γ und der Wärmeleitfähigkeit λ mittels Korrelation eine gesuchte physikalische Eigenschaft oder brenntechnisch relevante Grösse ermittelt.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise werden zuerst das Einlassventil 3 und Auslassventil 5 geöffnet, um das zu messende Gas oder Gasgemisch aus der Gashauptleitung 1 über die Messleitung 2 durch die Messvorrichtung fliessen zu lassen, womit sichergegangen werden kann, dass sich in der Messvorrichtung kein Fremdgas der letzten Messung mehr befindet. Das Einlassventil und Auslassventil können über eine Steuereinheit geöffnet werden. Fallweise kann auch die Auswerteinheit 11, wie in Fig. 1a gezeigt, die Steuerung des Einlassventils und Auslassventils übernehmen. Dann wird das Auslassventil 5 geschlossen und das Gasreservoir 4, dessen Volumeninhalt $V$ bekannt ist, füllt sich, bis das Einlassventil 3 geschlossen wird. Druck $p$ und Temperatur $T$ im Gasreservoir können mit dem Drucksensor 8 bzw. Temperatursensor 9 gemessen werden, sodass jederzeit auf das Normvolumen $V_{norm}$ des sich im Gasreservoir 4 befindlichen Gases oder Gasgemisches geschlossen werden kann.

$$V_{norm} = \frac{p}{1013.25\ mbar} \cdot \frac{273.15\ K}{T} \cdot V\ . \qquad (17)$$

Ist der Druck $p$ im Gasreservoir 4 grösser als der Druck $p_{krit}$, der benötigt wird, damit die Düse 6 kritisch betrieben werden kann, kann das Auslassventil 5 wieder geöffnet werden. Vorzugsweise ist der Druck $p$ im Gasreservoir um mehrere bar höher als $p_{krit}$, sodass die Druckabfallmessung über diesen Bereich der Drucküberhöhung vollzogen werden kann, ohne dass die Düse 6 nicht mehr kritisch betrieben ist. Jetzt wird das Auslassventil 5 wieder geschlossen, womit die Druckabfallmessung beendet ist. Der Drucksensor 8 ist vorzugsweise als Differenzdrucksensor gegenüber dem Auslass 10 der Messvorrichtung ausgelegt. Es ist jedoch auch möglich, einen zusätzlichen Drucksensor 8' am Auslass vorzusehen.

Während der Druckabfallmessung ist der zeitabhängige Druck $p(t)$ und die zeitabhängige Temperatur $T(t)$ im Druckreservoir 4 gemessen und von der Auswerteeinheit 11 aufgezeichnet worden. Mit diesen Daten wird in der Auswerteeinheit die Zeitkonstante $\tau$ in Gleichung (6) bzw. der Gaseigenschaftsfaktor Γ* in Gleichung (7) bestimmt. Gleichzeitig sind mit dem mikrothermischen Sensor 7 Durchflussdaten gemessen worden, die wiederum die Auswerteeinheit aufgezeichnet hat, um den Faktor $S$ in Gleichung (9) bzw. den Gaseigenschaftsfaktor Γ in Gleichung (11) zu bestimmen. Da Ein- und Auslassventil nach der Druckabfallmessung geschlossen sind, fliesst kein Gas mehr über den mikrothermischen Sensor 7. Jetzt kann die Messung der Wärmeleitfähigkeitsmessung λ erfolgen. Wiederum von der Auswerteeinheit aufgezeichnet, wird die Wärmeleitfähigkeit λ mit Hilfe der Lösung von Gleichung (12) bestimmt.

In der Auswerteeinheit 11 erfolgt nun die (fakultative) Validierung des Gaseigenschaftsfaktors Γ bzw. Γ* und danach, je nach gewünschter, brenntechnisch relevanter Grösse Q, deren Berechnung anhand der Gleichung (15) mit zuvor ermittelter Korrelationsfunktion $Q_{corr} = f_{corr}$ (Γ, Γ*, λ).

Bei Bedarf kann zudem, wie in Fig. 1b gezeigt, ein Verdichter 12' vorgesehen sein, der beispielsweise einlassseitig des Gasreservoirs 4 angeordnet ist, um den Druck im Gasreservoir zu erhöhen.

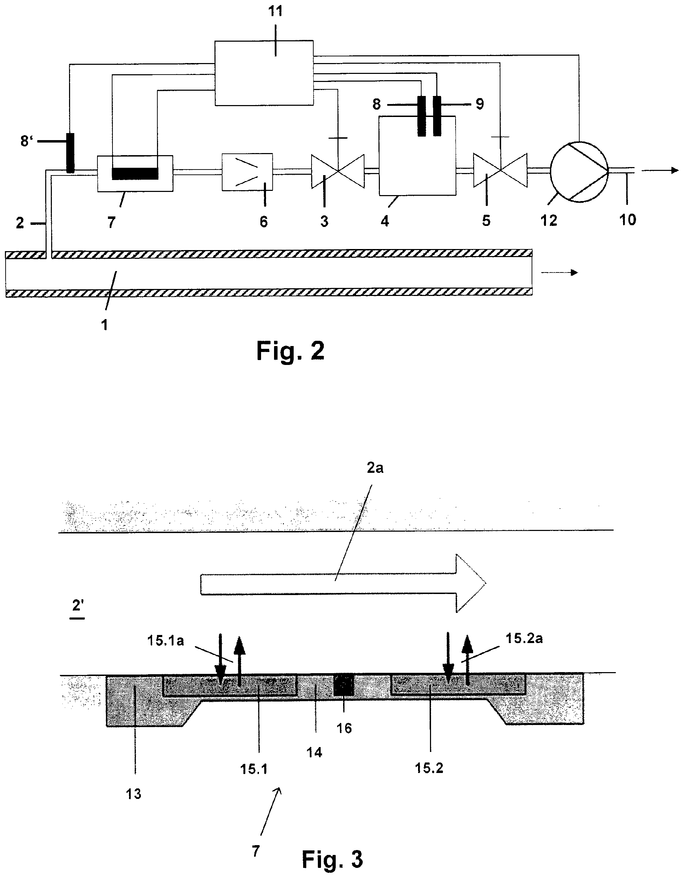

Fig. 2 zeigt ein zweites Ausführungsbeispiel des schematischen Aufbaus einer Messvorrichtung gemäss vorliegender Erfindung, in dem mit einem Unterdruck im Gasreservoir gearbeitet wird. Diese sogenannte Niederdruckvariante ist zum Beispiel vorteilhaft bei der Gasversorgung an Endkunden. Im zweiten Ausführungsbeispiel umfasst die Messvorrichtung zusätzlich zum Gasreservoir 4 einen Drucksensor 8, mit dem das Gasreservoir versehen ist, eine Auswerteeinheit 11, die zur Ausführung eines Verfahrens gemäss vorliegender Erfindung eingerichtet ist, eine kritische Düse 6, und einen mikrothermischen Sensor 7 zur Messung des Durchflusses und der Wärmeleitfähigkeit, wobei das Gasreservoir 4 für die Messung mit der kritischen Düse 6 und dem mikrothermischen Sensor 7 verbunden ist.

Bei Bedarf kann die Messvorrichtung zusätzlich eine oder mehrere der folgenden Komponenten enthalten: eine Vakuumpumpe 12, die mit dem Gasreservoir 4 verbunden ist, um einen Unterdruck im Gasreservoir zu erzeugen, eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der in der Messleitung 2 oder Gashauptleitung angeordnet sein kann, und einen Temperatursensor 9, der im Gasreservoir 4 angeordnet ist.

Ein Ausführungsbeispiel des Verfahrens zur Bestimmung physikalischer Eigenschaften und/oder brenntechnisch relevanter Grössen eines Gases und Gasgemisches gemäss vorliegender Erfindung wird im Folgenden anhand von Fig. 2 beschrieben. In dem Verfahren fliesst das Gas oder Gasgemisch unter Druck durch die kritische Düse 6 und über den mikrothermischen Sensor 7 in das Gasreservoir 4, wobei die kritische Düse und der mikrothermischen Sensor mit demselben Massenfluss beaufschlagt werden. Die Druckzunahme im Gasreservoir 4 wird als Funktion der Zeit gemessen und aus den Messwerten der Druckzunahme ein erster, von einer ersten Gruppe von physikalischen Eigenschaften des Gases oder Gasgemisches abhängiger Gaseigenschaftsfaktor $\Gamma^*$ bestimmt, wobei der erste Gaseigenschaftsfaktor zum Beispiel aus einer Proportionalitätskonstante der Druckzunahme abgeleitet wird. Aus dem Durchflusssignal des mikrothermischen Sensors 7 wird ein zweiter, von einer zweiten Gruppe von physikalischen Eigenschaften des Gases oder Gasgemisches abhängiger Gaseigenschaftsfaktor r bestimmt, wobei der zweite Gaseigenschaftsfaktor zum Beispiel die Wärmekapazität $c_p$ des Gases oder Gasgemisches enthält oder von derselben abhängig ist. Weiter wird mit Hilfe des mikrothermischen Sensors 7 die Wärmeleitfähigkeit $\lambda$ des Gases oder Gasgemisches bestimmt und aus dem ersten und/oder zweiten Gaseigenschaftsfaktor $\Gamma^*$, $\Gamma$ und der Wärmeleitfähigkeit $\lambda$ mittels Korrelation eine gesuchte physikalische Eigenschaft oder brenntechnisch relevante Grösse ermittelt.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise wird der Druck im Gasreservoir 4 vorgängig soweit erniedrigt, zum Beispiel mit einer Vakuumpumpe 12, dass die kritische Düse 6 kritisch betrieben werden kann, d.h. der Druck im Gasreservoir weniger als die Hälfte des Drucks vor der kritischen Düse beträgt. Es ist kein Hochvakuum erforderlich: Solang der Druck $p$ und die Temperatur $T$ im Gasreservoir 4 gemessen werden, kann immer berechnet werden, welches Normvolumen an Gas in das Gasreservoir geflossen ist. Es ist jedoch von Vorteil, wenn der Druck um einen Faktor niedriger ist als es für kritische Verhältnisse nötig wäre, da dann entsprechend länger gemessen werden kann, was eine genauere Bestimmung der Proportionalitätskonstante ermöglicht.

Für weitere Einzelheiten zum Verfahren, die bei Bedarf verwendet werden können, wird auf die Beschreibung des ersten Ausführungsbeispiels verwiesen, wobei gegebenenfalls der Begriff "Druckabfall" durch "Druckzunahme" ersetzt werden muss.

Fig. 3 zeigt ein Ausführungsbeispiel eines mikrothermischen Sensors zur Verwendung in einer Messvorrichtung gemäss vorliegender Erfindung. Der mikrothermische Sensor 7 kann beispielsweise, wie in Fig. 3 gezeigt, ein integriertes, mikrothermisches CMOS-Hitzdrahtanemometer sein, das im Einsatz in einem Abschnitt 2' der Messleitung angeordnet und mit einem Gas- oder Gasgemischstrom 2a beaufschlag werden kann. Das mikrothermische CMOS-Hitzdrahtanemometer umfasst ein Substrat 13, das typisch eine Membran 14 von wenigen Mikrometer Dicke enthält. Weiter umfasst das CMOS-Hitzdrahtanemometer zwei Thermoelemente 15.1, 15.2 und ein Heizelement 16, das in Flussrichtung zwischen den beiden Thermoelementen angeordnet sein kann. Mit den beiden Thermoelementen 15.1, 15.2 kann die Temperatur erfasst werden, die sich auf Grund des Wärmeaustausches 15.1a, 15.2a mit dem Gas- oder Gasgemischstrom 2a einstellt.

Für weitere Einzelheiten zur Funktionsweise des integrierten, mikrothermischen CMOS-Hitzdrahtanemometers wird auf D. Matter, B. Kramer, T. Kleiner, B. Sabbattini, T. Suter, "Mikroelektronischer Haushaltsgaszähler mit neuer Technologie", Technisches Messen 71, 3 (2004), S. 137-146 verwiesen.

Fig. 4 zeigt eine Darstellung des direkt gemessenen Dichteverhältnisses $\rho / \rho_{ref}$ (y-Achse) in Abhängigkeit des korrelierten Dichteverhältnisses $\rho_{corr} / \rho_{ref}$ (x-Achse) für verschieden Gasgruppen bei Normbedingungen (0°C, 1013.25 mbar), wobei das korrelierte Dichteverhältnisse mit einem Verfahren beziehungsweise mit einer Messvorrichtung gemäss vorliegender Erfindung ermittelt wurde. Als Referenzgas wurde ein typisches H-Erdgas verwendet.

Die oben stehend beschriebene Messvorrichtung zur Bestimmung physikalischer Eigenschaften und/oder brenntechnisch relevanter Grössen eines Gases und Gasgemisches ist einer neuen Kategorie zuzuteilen, nämlich "Messung des Druckabfalls oder der Druckzunahme in einem Gasreservoir, wobei das Gas durch eine kritische Düse fliesst, sowie Wärmeleitfähigkeits- und Durchflussmessung mit Hilfe eines mikrothermischen Sensors, und Datenvalidierung durch Aufsummieren der Durchflusswerte". Die verwendeten Komponenten sind kostengünstig, wodurch neue Märkte erschlossen werden können, in denen heute aus Kostengründen keine Gasqualitätssensoren eingesetzt werden. Von der Genauigkeit her sind nur wenig Einbussen gegenüber teureren, kommerziell erhältlichen Geräten zu erwarten, da hier ebenfalls mindestens drei voneinander unabhängige Messwerte für die Korrelation verwendet werden.

Weiter umfasst die Erfindung in einer zweiten Ausführungsform die Verwendung eines Gasreservoirs und einer kritischen Düse zur Bestimmung physikalischer Eigenschaften und/oder brenntechnisch relevanter Grössen eines Gases oder Gasgemisches, bzw. ein Verfahren, in dem ein Gasreservoir und eine kritische Düse zur Bestimmung physikalischer Eigenschaften und/oder brenntechnisch relevanter Grössen eines Gases oder Gasgemisches verwendet werden, wobei das Gas oder Gasgemisch unter Druck aus dem Gasreservoir durch die kritische Düse fliesst, wobei der Druckabfall im Gasreservoir als Funktion der Zeit gemessen wird, aus den Messwerten des Druckabfalls ein von physikalischen Eigenschaften des Gases oder Gasgemisches abhängiger Gaseigenschaftsfaktor $\Gamma^*$ bestimmt wird, der zum Beispiel aus einer Zeitkonstante des Druckabfalls abgeleitet wird, und aus dem Gaseigenschaftsfaktor $\Gamma^*$ mittels Korrelation eine gesuchte physikalische Eigenschaft oder brenntechnisch relevante Grösse ermittelt wird.

Die oben beschriebene zweite Ausführungsform der Erfindung kann auch als eigenständige Erfindung betrachtet werden.

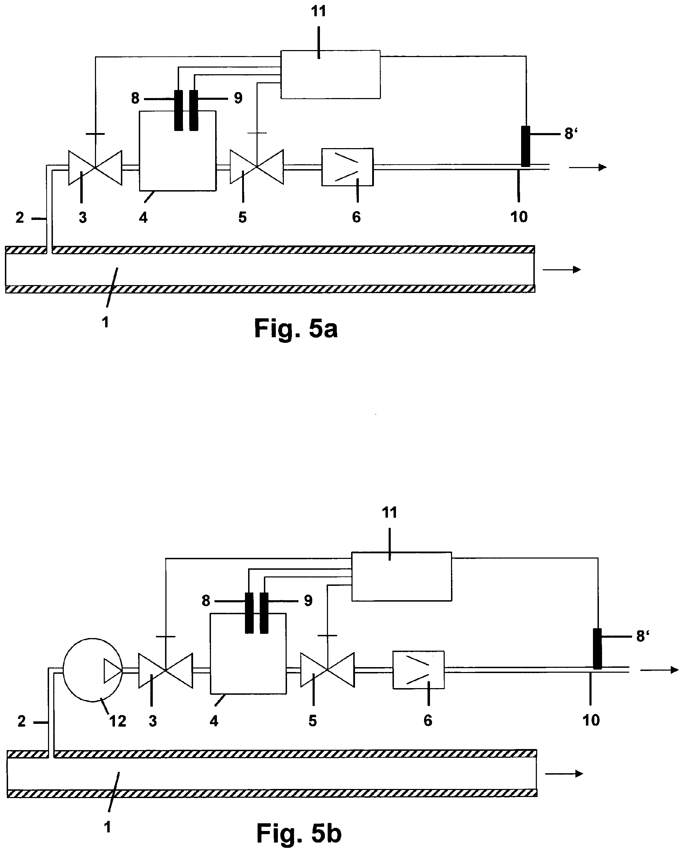

Fig. 5a zeigt ein Ausführungsbeispiel des schematischen Aufbaus einer Messvorrichtung gemäss der zweiten Ausführungsform der Erfindung im Fall, in dem die Gashauptleitung 1 einen Druck aufweist, der höher als der kritische Druck für die kritische Düse 6 der Messvorrichtung ist (Hochdruckvariante). Im Ausführungsbeispiel umfasst die Messvorrichtung zusätzlich zur kritischen Düse 6 eine Auswerteeinheit 11, die zur Ausführung eines Verfahrens gemäss der zweiten Ausführungsform der Erfindung eingerichtet ist, und ein Gasreservoir 4, das mit einem Drucksensor 8 versehen ist, wobei das Gasreservoir 4 für die Messung mit der kritischen Düse 6 verbunden ist.

Bei Bedarf kann die Messvorrichtung zusätzlich eine oder mehrere der folgenden Komponenten enthalten: eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der am Auslass 10 angeordnet sein kann, einen Temperatursensor 9, der im Gasreservoir angeordnet ist, und einen Verdichter 12', der einlassseitig des Gasreservoirs 4 angeordnet sein kann, um den Druck im Gasreservoir zu erhöhen.

Ein Ausführungsbeispiel des Verfahrens zur Bestimmung physikalischer Eigenschaften und/oder brenntechnisch relevanter Grössen eines Gases oder Gasgemisches gemäss der zweiten Ausführungsform der Erfindung wird im Folgenden anhand von Fig. 5a beschrieben. In diesem Ausführungsbeispiel fliesst das Gas oder Gasgemisch aus dem Gasreservoir 4 durch die kritische Düse 6. Der Druckabfall im Gasreservoir 4 wird als Funktion der Zeit gemessen und aus den Messwerten des Druckabfalls ein von einer Gruppe von physikalischen Eigenschaften des Gases oder Gasgemisches abhängiger Gaseigenschaftsfaktor $\Gamma^*$ bestimmt, wobei der Gaseigenschaftsfaktor zum Beispiel aus einer Zeitkonstante des Druckabfalls abgeleitet wird. Weiter wird aus dem Gaseigenschaftsfaktor $\Gamma^*$ mittels Korrelation eine gesuchte physikalische Eigenschaft oder brenntechnisch relevante Grösse ermittelt.

Vorteilhafterweise werden mit der zweiten Ausführungsform der Erfindung binäre Gasgemische auf ihren Anteil der beiden das Gasgemisch bildenden Komponenten analysiert, da der Gaseigenschaftsfaktor $\Gamma^*$ intrinsisch eine stetige Funktion der Gasanteile x% bzw. (1-x%) ist. Mit Kenntnis des Anteil x% bzw. (1-x%) können anschliessend aus Tabellenwerken oder mittels entsprechenden Berechnungsprogrammen physikalische Eigenschaften und/oder brenntechnisch relevanter Grösse des binären Gasgemisches bestimmt werden. Natürlich ist auch die direkte Korrelation dieser physikalische Eigenschaften und/oder brenntechnisch relevanten Grösse des binären Gasgemisches mit dem Gaseigenschaftsfaktor $\Gamma^*$ möglich.

In einer Ausführungsvariante des Verfahrens wird somit der prozentuale Anteil der einen Komponente in einem binären Gasgemisch bestimmt, wobei die zu korrelierende Grösse entweder dem Zusammensetzungsanteil der einen Komponente (x%) und/oder einer beliebig anderen physikalische Eigenschaft des binären Gasgemisches entspricht.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise werden zuerst das Einlassventil 3 und Auslassventil 5 geöffnet, um das zu messende Gas oder Gasgemisch aus der Gashauptleitung 1 über die Messleitung 2 durch die Messvorrichtung fliessen zu lassen, womit sichergegangen werden kann, dass sich in der Messvorrichtung kein Fremdgas der letzten Messung mehr befindet. Das Einlassventil und Auslassventil können über eine Steuereinheit geöffnet werden. Fallweise kann auch die Auswerteeinheit 11, wie in Fig. 5a gezeigt, die Steuerung des Einlassventils und Auslassventils übernehmen. Dann wird das Auslassventil 5 geschlossen und das Gasreservoir 4, dessen Volumeninhalt $V$ bekannt ist, füllt sich, bis das Einlassventil 3 geschlossen wird. Druck $p$ und Temperatur $T$ im Gasreservoir können mit dem Drucksensor 8 bzw. Temperatursensor 9 gemessen werden, sodass jederzeit auf das Normvolumen $V_{norm}$ des sich im Gasreservoir 4 befindlichen Gases oder Gasgemisches geschlossen werden kann.

$$V_{norm} \;=\; \frac{p}{1013.25\,mbar} \cdot \frac{273.15\,K}{T} \cdot V \quad . \qquad (17)$$

Ist der Druck $p$ im Gasreservoir 4 grösser als der Druck $p_{krit}$, der benötigt wird, damit die Düse 6 kritisch betrieben werden kann, kann das Auslassventil 5 wieder geöffnet werden. Vorzugsweise ist der Druck $p$ im Gasreservoir um mehrere bar höher als $p_{krit}$, sodass die Druckabfallmessung über diesen Bereich der Drucküberhöhung vollzogen werden kann, ohne dass die Düse 6 nicht mehr kritisch betrieben ist. Jetzt wird das Auslassventil 5 wieder geschlossen, womit die Druckabfallmessung beendet ist. Der Drucksensor 8 ist vorzugsweise als Differenzdrucksensor gegenüber dem Auslass 10 der Messvorrichtung ausgelegt. Es ist jedoch auch möglich, einen zusätzlichen Drucksensor 8' am Auslass vorzusehen.

Während der Druckabfallmessung ist der zeitabhängige Druck $p(t)$ und die zeitabhängige Temperatur $T(t)$ im Druckreservoir 4 gemessen und von der Auswerteeinheit 11 aufgezeichnet worden. Mit diesen Daten werden in der Auswerteeinheit die Zeitkonstante $\tau$ in Gleichung (6) bzw. die Proportionalitätskonstante in Gleichung (6') und der Gaseigenschaftsfaktor $\Gamma^*$ in Gleichung (7) bzw. (7')bestimmt.

In der Auswerteeinheit 11 erfolgt nun, je nach gewünschter, brenntechnisch relevanter Grösse Q, deren Berechnung anhand der Gleichung (15) mit zuvor ermittelter Korrelationsfunktion $Q_{corr} = f_{corr}(\Gamma^*)$.

Bei Bedarf kann zudem, wie in Fig. 5b gezeigt, ein Verdichter 12' vorgesehen sein, der beispielsweise einlassseitig des Gasreservoirs 4 angeordnet ist, um den Druck im Gasreservoir zu erhöhen.

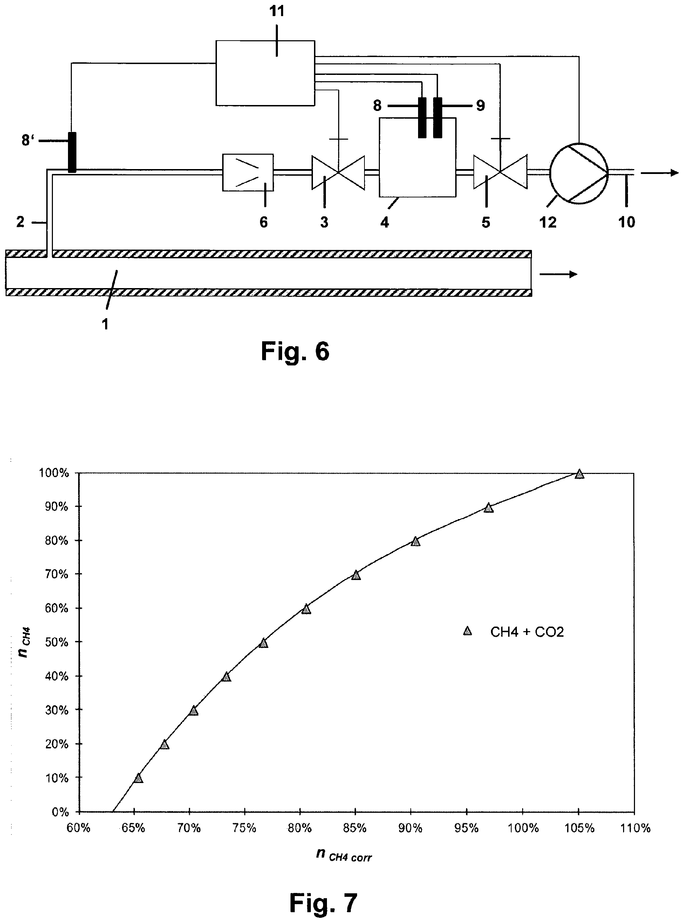

Fig. 6 zeigt ein zweites Ausführungsbeispiel des schematischen Aufbaus einer Messvorrichtung gemäss der zweiten Ausführungsform der Erfindung, in dem mit einem Unterdruck im Gasreservoir gearbeitet wird. Diese sogenannte Niederdruckvariante ist zum Beispiel vorteilhaft bei der Gasversorgung an Endkunden. Im zweiten Ausführungsbeispiel umfasst die Messvorrichtung zusätzlich zum Gasreservoir 4 einen Drucksensor 8, mit dem das Gasreservoir versehen ist, eine Auswerteeinheit 11, die zur Ausführung eines Verfahrens gemäss der zweiten Ausführungsform der Erfindung eingerichtet ist, und eine kritische Düse 6, wobei das Gasreservoir 4 für die Messung mit der kritischen Düse 6 verbunden ist.

Bei Bedarf kann die Messvorrichtung zusätzlich eine oder mehrere der folgenden Komponenten enthalten: eine Vakuumpumpe 12, die mit dem Gasreservoir 4 verbunden ist, um einen Unterdruck im Gasreservoir zu erzeugen, eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der in der Messleitung 2 oder Gashauptleitung angeordnet sein kann, und einen Temperatursensor 9, der im Gasreservoir 4 angeordnet ist.

Ein weiteres Ausführungsbeispiel des Verfahrens zur Bestimmung physikalischer Eigenschaften und/oder brenntechnisch relevanter Grössen eines Gases und Gasgemisches gemäss der zweiten Ausführungsform der Erfindung wird im Folgenden anhand von Fig. 6 beschrieben. In diesem Ausführungsbeispiel fliesst das Gas oder Gasgemisch unter Druck durch die kritische Düse 6 in das Gasreservoir 4. Die Druckzunahme im Gasreservoir 4 wird als Funktion der Zeit gemessen und aus den Messwerten der Druckzunahme ein von einer Gruppe von physikalischen Eigenschaften des Gases oder Gasgemisches abhängiger Gaseigenschaftsfaktor $\Gamma^*$ bestimmt, wobei der Gaseigenschaftsfaktor zum Beispiel aus einer Proportionalitätskonstante der Druckzunahme abgeleitet wird. Aus dem Gaseigenschaftsfaktor $\Gamma^*$ wird mittels Korrelation eine gesuchte physikalische Eigenschaft oder brenntechnisch relevante Grösse ermittelt.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise wird der Druck im Gasreservoir 4 vorgängig soweit erniedrigt, zum Beispiel mit einer Vakuumpumpe 12, dass die kritische Düse 6 kritisch betrieben werden kann, d.h. der Druck im Gasreservoir weniger als die Hälfte des Drucks vor der kritischen Düse beträgt. Es ist kein Hochvakuum erforderlich: Solang der Druck $p$ und die Temperatur $T$ im Gasreservoir 4 gemessen werden, kann immer berechnet werden, welches Normvolumen an Gas in das Gasreservoir geflossen ist. Es ist jedoch von Vorteil, wenn der Druck um einen Faktor niedriger ist als es für kritische Verhältnisse nötig wäre, da dann entsprechend länger gemessen werden kann, was eine genauere Bestimmung der Proportionalitätskonstante ermöglicht.

Für weitere Einzelheiten zum Verfahren, die bei Bedarf verwendet werden können, wird auf die Beschreibung des ersten Ausführungsbeispiels verwiesen, wobei gegebenenfalls der Begriff "Druckabfall" durch "Druckzunahme" ersetzt werden muss.

Fig. 7 zeigt eine Darstellung des direkt gemessenen Methananteils $n_{cH4}$ (y-Achse) in Abhängigkeit des korrelierten Methananteils $n_{CH4\ corr}$ (x-Achse) für ein aus Methan und Kohlendioxid bestehendes, binäres Roh-Biogas bei Normbedingungen (0°C, 1013.25 mbar), wobei der korrelierte Methananteil mit einem Verfahren beziehungsweise mit einer Messvorrichtung gemäss der zweiten Ausführungsform der Erfindung ermittelt wurde. Als Referenzgas wurde ein typisches H-Erdgas verwendet. Die gesuchte Grösse Q (hier der Methananteil $n_{CH4\ corr}$ in x%) wird vorteilhafterweise mittels der Korrelationsfunktion $Q_{corr} = a + b \cdot \Gamma^* + c \cdot \Gamma^{*2} + d \cdot \Gamma^{*3}$ ermittelt, wobei in dem in der Darstellung gezeigten Beispiel numerisch a = -7.82, b = 22.7, c = -20.4 und d = 6.45.

Die oben stehend beschriebene Messvorrichtung zur Bestimmung physikalischer Eigenschaften und/oder brenntechnisch relevanter Grössen eines Gases und Gasgemisches ist einer neuen Kategorie zuzuteilen, nämlich "Messung des Druckabfalls oder der Druckzunahme in einem Gasreservoir, wobei das Gas durch eine kritische Düse fliesst. Die verwendeten Komponenten sind kostengünstig, wodurch neue Märkte erschlossen werden können, in denen heute aus Kostengründen keine Gasqualitätssensoren eingesetzt werden. Von der Genauigkeit her sind gewisse Einbussen gegenüber teureren, kommerziell erhältlichen Geräten zu erwarten, da hier anstatt drei nur ein unabhängiger Messwert für die Korrelation verwendet wird.

Zusätzlich umfasst die Erfindung in einer dritten Ausführungsform die Verwendung eines Gasreservoirs und eines für ein bestimmtes Kalibriergas oder -Gasgemisch kalibrierten mikrothermischen Sensors zur Bestimmung physikalischer Eigenschaften und/oder brenntechnisch relevanter Grössen eines Gases oder Gasgemisches, bzw. ein Verfahren, in dem ein Gasreservoir und ein für ein bestimmtes Kalibriergas oder -Gasgemisch kalibrierter mikrothermischer Sensor zur Bestimmung physikalischer Eigenschaften und/oder brenntechnisch relevanter Grössen eines Gases oder Gasgemisches verwendet werden, wobei das Gas oder Gasgemisch unter Druck aus dem Gasreservoir über den mikrothermischen Sensor fliesst, wobei der mit dem für ein bestimmtes Kalibriergas oder -Gasgemisch kalibrierte mikrothermische Sensor bestimmte Volumenfluss $v_x \cdot A$ aufsummiert und mit dem aus dem Gasreservoir ausgeflossenen Gasvolumen verglichen wird, aus dem Vergleich der beiden Volumina ein von physikalischen Eigenschaften des Gases oder Gasgemisches abhängiger Gaseigenschaftsfaktor $S / v'_x$ bestimmt wird, in dem $v'_x$ die aus dem ausgeflossenen Gasvolumen bestimmte Fliessgeschwindigkeit bezeichnet, und aus dem Gaseigenschaftsfaktor, der zum Beispiel durch $S / v'_x = c_p \cdot \rho / \lambda$ gegeben sein kann (siehe Gleichung (9)), mittels Korrelation eine gesuchte physikalische Eigenschaft oder brenntechnisch relevante Grösse ermittelt wird.

Die oben beschriebene dritte Ausführungsform der Erfindung kann auch als eigenständige Erfindung betrachtet werden.

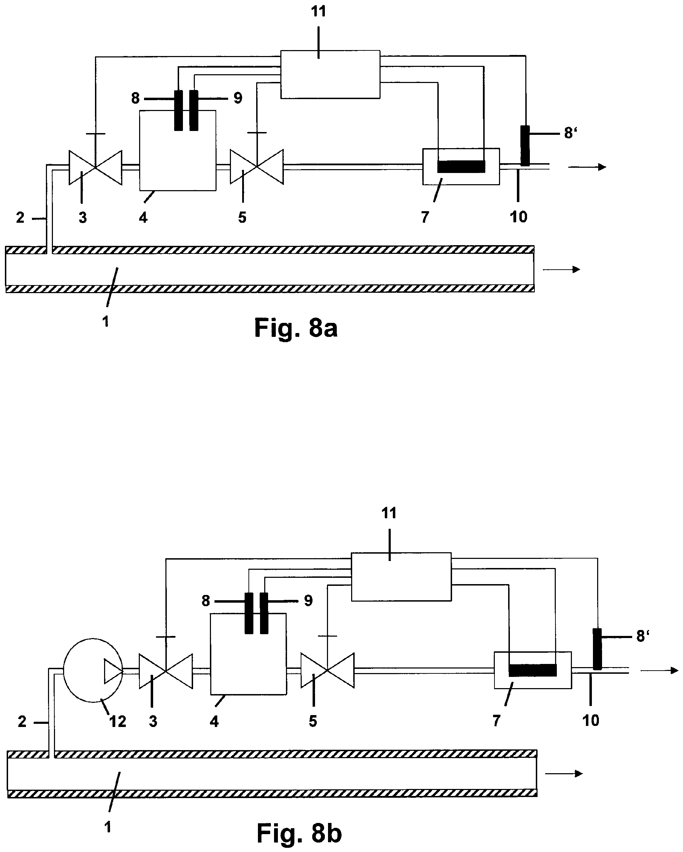

Fig. 8a zeigt ein Ausführungsbeispiel des schematischen Aufbaus einer Messvorrichtung gemäss der dritten Ausführungsform der Erfindung im Fall, in dem die Gashauptleitung 1 unter Druck steht (Hochdruckvariante). Im Ausführungsbeispiel umfasst die Messvorrichtung eine Auswerteeinheit 11, die zur Ausführung eines Verfahrens gemäss der dritten Ausführungsform der Erfindung eingerichtet ist, ein Gasreservoir 4, das mit einem Drucksensor 8 versehen ist, und einen mikrothermischen Sensor 7 zur Messung des Durchflusses und der Wärmeleitfähigkeit, wobei das Gasreservoir 4 für die Messung mit dem mikrothermischen Sensor 7 verbunden ist.

Bei Bedarf kann die Messvorrichtung zusätzlich eine oder mehrere der folgenden Komponenten enthalten: eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der am Auslass 10 angeordnet sein kann, einen Temperatursensor 9, der im Gasreservoir angeordnet ist, und einen Verdichter 12', der einlassseitig des Gasreservoirs 4 angeordnet sein kann, um den Druck im Gasreservoir zu erhöhen.

Ein Ausführungsbeispiel des Verfahrens zur Bestimmung physikalischer Eigenschaften und/oder brenntechnisch relevanter Grössen eines Gases und Gasgemisches gemäss der dritten Ausführungsform der Erfindung wird im Folgenden anhand von Fig. 8a beschrieben. In dem Verfahren fliesst das Gas oder Gasgemisch unter Druck aus dem Gasreservoir 4 über den für ein bestimmtes Kalibriergas oder -Gasgemisch kalibrierten mikrothermischen Sensor 7, wobei der Volumenfluss $v_x \cdot A$ aufsummiert und mit dem aus dem Gasreservoir ausgeflossenen Gasvolumen verglichen wird, aus dem Vergleich der beiden Volumina ein von physikalischen Eigenschaften des Gases oder Gasgemisches abhängiger Gaseigenschaftsfaktor $S / v'_x$ bestimmt wird, in dem $v'_x$ die aus dem ausgeflossenen Gasvolumen bestimmte Fliessgeschwindigkeit bezeichnet, und aus dem Gaseigenschaftsfaktor, der zum Beispiel durch $S / v'_x = c_p \cdot \rho / \lambda$ gegeben sein kann (siehe Gleichung (9)), mittels Korrelation eine gesuchte physikalische Eigenschaft oder brenntechnisch relevante Grösse ermittelt wird.

In einer vorteilhaften Ausführungsform des Verfahrens wird mit Hilfe des mikrothermischen Sensors 7 zusätzlich die Wärmeleitfähigkeit $\lambda$ des Gases oder Gasgemisches bestimmt.

Vorteilhafterweise werden mit der dritten Ausführungsform der Erfindung Erdgasgemische auf ihre Zugehörigkeit zu den H-Gasen bzw. L-Gasen untersucht (Gase mit hohem (High) bzw. niedrigem (Low) Brennwert), da der Gaseigenschaftsfaktor, der zum Beispiel durch $S / v'_x = c_p \cdot \rho / \lambda$ gegeben sein kann (siehe Gleichung (9)), dem Kehrwert der Wärmediffusivität des Gasgemisches entspricht, anhand derer zusammen mit der Wärmeleitfähigkeit $\lambda$, die mit dem mikrothermischen Sensor separat gemessen werden kann, eine Unterscheidung der H- bzw. L-Gasgruppe möglich wird.

Die Klassenzugehörigkeit eines Erdgasgemisches zur H- bzw. L-Gas Gruppe kann beispielsweise bestimmt werden, indem der Gaseigenschaftsfaktor ($S / v'_x$) mit dem Kehrwert der Wärmediffusivität $c_p \cdot \rho / \lambda$ identifiziert wird, und indem bei gegebener Wärmeleitfähigkeit die Zuteilung anhand eines Grenzwerts für die Wärmediffusivität erfolgt, oberhalb dessen ein Gasgemisch als L-Gas und unterhalb dessen als H-Gas klassifiziert wird.

In einer Ausführungsvariante des Verfahrens wird somit mit Hilfe des mikrothermischen Sensors 7 zusätzlich die Wärmeleitfähigkeit $\lambda$ des Gases oder Gasgemisches bestimmt und zusammen mit dem Gaseigenschaftsfaktors $S / v'_x = c_p \cdot \rho / \lambda$ eine Einteilung des gemessenen Gases in H- beziehungsweise L-Gas vorgenommen.

Weitere vorteilhafte Ausführungsformen und -varianten des Verfahrens finden sich in vorangehenden Abschnitten der Beschreibung. Die nachstehende Beschreibung enthält zusätzliche Einzelheiten zum Verfahren, die bei Bedarf verwendet werden können.

Vorteilhafterweise werden zuerst das Einlassventil 3 und Auslassventil 5 geöffnet, um das zu messende Gas oder Gasgemisch aus der Gashauptleitung 1 über die Messleitung 2 durch die Messvorrichtung fliessen zu lassen, womit sichergegangen werden kann, dass sich in der Messvorrichtung kein Fremdgas der letzten Messung mehr befindet.

Das Einlassventil und Auslassventil können über eine Steuereinheit geöffnet werden. Fallweise kann auch die Auswerteeinheit 11, wie in Fig. 8a gezeigt, die Steuerung des Einlassventils und Auslassventils übernehmen. Dann wird das Auslassventil 5 geschlossen und das Gasreservoir 4, dessen Volumeninhalt $V$ bekannt ist, füllt sich, bis das Einlassventil 3 geschlossen wird. Druck $p$ und Temperatur $T$ im Gasreservoir können mit dem Drucksensor 8 bzw. Temperatursensor 9 gemessen werden, sodass jederzeit auf das Normvolumen $V_{norm}$ des sich im Gasreservoir 4 befindlichen Gases oder Gasgemisches geschlossen werden kann.

$$V_{norm} \ = \ \frac{p}{1013.25\,mbar} \cdot \frac{273.15\,K}{T} \cdot V \ . \qquad (17)$$

Nun kann das Auslassventil 5 wieder geöffnet werden. Vorzugsweise ist der Druck $p$ im Gasreservoir 4 um soviel höher als der Druck nach dem Gasreservoir, dass die Zeitspanne, in der das Gas aus dem Gasreservoir 4 über den mikrothermischen Sensor 7 fliesst, lange genug ist, um den Volumenfluss $v_x \cdot A$ genügend genau aufsummieren zu können. Jetzt wird das Auslassventil 5 wieder geschlossen, womit die Durchflussmessung beendet ist. Der Drucksensor 8 ist vorzugsweise als Differenzdrucksensor gegenüber dem Auslass 10 der Messvorrichtung ausgelegt. Es ist jedoch auch möglich, einen zusätzlichen Drucksensor 8' am Auslass vorzusehen.

Während der Durchflussmessung sind mit dem mikrothermischen Sensor 7 Durchflussdaten gemessen und von der Auswerteeinheit 11 aufgezeichnet worden, um den Faktor $S$ in Gleichung (9) zu bestimmen. Da Ein- und Auslassventil nach der Durchflussmessung geschlossen sind, fliesst kein Gas mehr über den mikrothermischen Sensor 7. Jetzt kann die Messung der Wärmeleitfähigkeitsmessung $\lambda$ erfolgen. Wiederum von der Auswerteeinheit aufgezeichnet, wird die Wärmeleitfähigkeit $\lambda$ mit Hilfe der Lösung von Gleichung (12) bestimmt.

Mit diesen Daten wird in der Auswerteeinheit 11 der Volumenfluss zum Volumen $V_{sum}$ aufsummiert und mit dem aus dem Gasreservoir ausgeflossenen Gasvolumen $V_{diff}$ verglichen. Aus dem Vergleich der beiden Volumina kann nun ein von physikalischen Eigenschaften des Gases oder Gasgemisches abhängiger Gaseigenschaftsfaktor $S / v'_x$ bestimmt wird, in dem $v'_x$ die aus dem ausgeflossenen Gasvolumen bestimmte Fliessgeschwindigkeit bezeichnet. Zweckmässigerweise werden die Volumina für den Vergleich mittels Gleichung (17) auf Normbedingungen umgerechnet, so dass $v'_x$ durch

$$v'_x \ = \ v_x \cdot V_{diff}^{norm} / V_{sum}^{norm} \qquad (18)$$

gegeben ist mit dem auf Normbedingungen umgerechneten, ausgeflossenen Gasvolumen $V_{diff}^{norm}$ und dem auf Normbedingungen umgerechneten, aufsummierten Volumen $V_{sum}^{norm}$. Danach, je nach gewünschter, brenntechnisch relevanter Grösse Q, erfolgt nun in der Auswerteeinheit 11 deren Berechnung anhand der Gleichung (15) mit zuvor ermittelter Korrelationsfunktion $Q_{corr} = f_{corr} (S / v'_x)$ oder der Wert von $S / v'_x$ wird dazu benutzt, um zusammen mit der Wärmeleitfähigkeit $\lambda$ ein Erdgasgemisch der Kategorie H- bzw. L-Gas zuzuordnen.

Bei Bedarf kann zudem, wie in Fig. 8b gezeigt, ein Verdichter 12' vorgesehen sein, der beispielsweise einlassseitig des Gasreservoirs 4 angeordnet ist, um den Druck im Gasreservoir zu erhöhen.

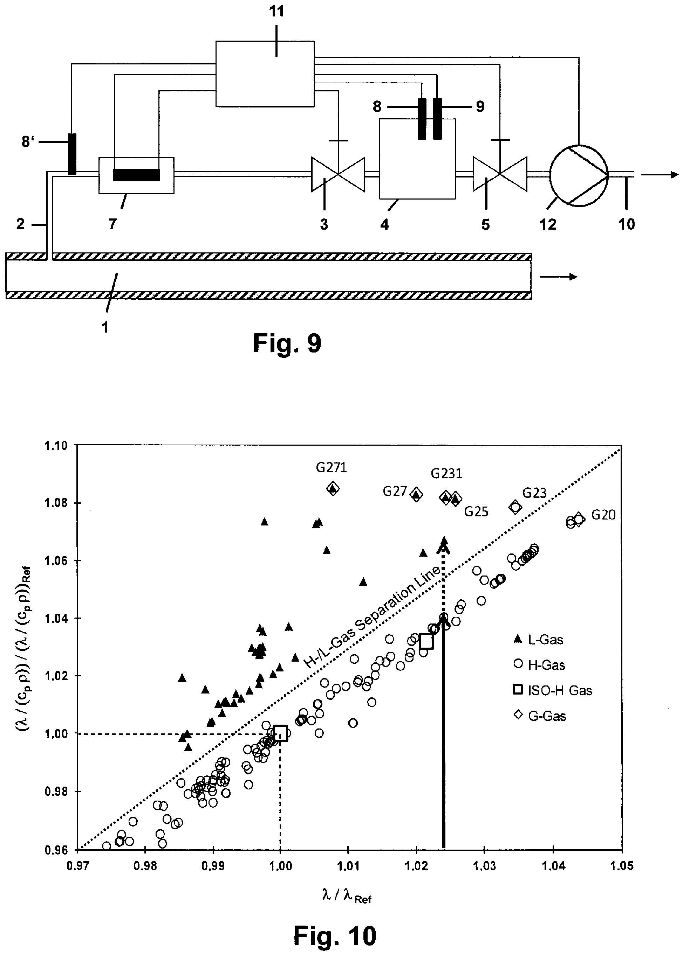

Fig. 9 zeigt ein zweites Ausführungsbeispiel des schematischen Aufbaus einer Messvorrichtung gemäss der dritten Ausführungsform der Erfindung, in dem mit einem Unterdruck im Gasreservoir gearbeitet wird. Diese sogenannte Niederdruckvariante ist zum Beispiel vorteilhaft bei der Gasversorgung an Endkunden. Im zweiten Ausführungsbeispiel umfasst die Messvorrichtung zusätzlich zum Gasreservoir 4 einen Drucksensor 8, mit dem das Gasreservoir versehen ist, eine Auswerteeinheit 11, die zur Ausführung eines Verfahrens gemäss der dritten Ausführungsform der Erfindung eingerichtet ist und einen mikrothermischen Sensor 7 zur Messung des Durchflusses und der Wärmeleitfähigkeit, wobei das Gasreservoir 4 für die Messung mit dem mikrothermischen Sensor 7 verbunden ist.

Bei Bedarf kann die Messvorrichtung zusätzlich eine oder mehrere der folgenden Komponenten enthalten: eine Vakuumpumpe 12, die mit dem Gasreservoir 4 verbunden ist, um einen Unterdruck im Gasreservoir zu erzeugen, eine Messleitung 2, die zum Gasreservoir 4 führt, und die im Betrieb mit einer Hauptgasleitung 1 verbunden sein kann, ein Einlassventil 3, das in der Messleitung 2 angeordnet sein kann, um die Gaszufuhr zum Gasreservoir zu steuern, ein Auslassventil 5, das ausgangsseitig des Gasreservoirs angeordnet ist, um den Gasfluss aus dem Gasreservoir zu steuern, einen Auslass 10, um das aus der Messvorrichtung ausfliessende Gas abzuführen, einen zusätzlichen Drucksensor 8', der in der Messleitung 2 oder Gashauptleitung angeordnet sein kann, und einen Temperatursensor 9, der im Gasreservoir 4 angeordnet ist.

Ein weiteres Ausführungsbeispiel des Verfahrens zur Bestimmung physikalischer Eigenschaften und/oder brenntechnisch relevanter Grössen eines Gases und Gasgemisches gemäss der dritten Ausführungsform der Erfindung wird im Folgenden anhand von Fig. 9 beschrieben. In diesem Ausführungsbeispiel fliesst das Gas oder Gasgemisch unter einem Druck, der typisch soviel höher als der Druck nach dem Gasreservoir ist, dass die Zeitspanne, in der das Gas aus dem Gasreservoir 4 über den mikrothermischen Sensor 7 fliesst, lange genug ist, um den Volumenfluss $v_x \cdot A$ genügend genau aufsummieren zu können. Der aufsummierte Volumenfluss $V_{sum}$ wird mit dem aus dem Gasreservoir ausgeflossenen Gasvolumen $V_{diff}$ verglichen, aus dem Vergleich der beiden Volumina ein von physikalischen Eigenschaften des Gases oder Gasgemisches abhängiger Gaseigenschaftsfaktor $S / v'_x$ bestimmt, in dem $v'_x$ die aus dem ausgeflossenen Gasvolumen bestimmte Fliessgeschwindigkeit bezeichnet, und aus dem Gaseigenschaftsfaktor, der zum Beispiel durch $S / v'_x = c_p \cdot \rho / \lambda$ gegeben sein kann (siehe Gleichung (9)), mittels Korrelation eine gesuchte physikalische Eigenschaft oder brenntechnisch relevante Grösse ermittelt.

In einer vorteilhaften Ausführungsform des Verfahrens wird mit Hilfe des mikrothermischen Sensors 7 die Wärmeleitfähigkeit $\lambda$ des Gases oder Gasgemisches bestimmt und beispielsweise zusammen mit dem Gaseigenschaftsfaktors $S / v'_x = c_p \cdot \rho / \lambda$ eine Einteilung des gemessenen Gases in H- beziehungsweise L-Gas vorgenommen.

Für weitere vorteilhafte Ausführungsformen und -varianten des Verfahrens und für Einzelheiten zum Verfahren, die bei Bedarf verwendet werden können, wird auf die vorangehenden Abschnitte der Beschreibung verwiesen, wobei gegebenenfalls der Begriff "Druckabfall" durch "Druckzunahme" ersetzt werden muss.

Fig. 10 zeigt eine Darstellung, wie anhand bekannter Wärmeleitfähigkeiten $\lambda$ (x-Achse) und Wärmediffusivitäten $\lambda / (c_p \rho)$, auch Temperaturleitfähigkeiten genannt, (y-Achse) eine Unterscheidung in H- bzw. L-Gas getroffen werden kann. L-Gase oberhalb der H-/L-Gas Trennlinie (H-/L-Gas Separation Line) haben typischerweise höhere Wärmediffusivitäten als H-Gase bei gleicher Wärmeleitfähigkeit unterhalb der Trennlinie (doppelter Pfeil bei x ≈1.024). Da der Gaseigenschaftsfaktor $S / v'_x = c_p \cdot \rho / \lambda$ im Wesentlichen der Kehrwert der Wärmediffusivität des Gasgemisches ist, kann demnach mit zusätzlich gemessener Wärmeleitfähigkeit $\lambda$ die Unterscheidung H- bzw. L-Gas getroffen werden. Alle Werte sind bei Normbedingungen (0°C, 1013.25 mbar) gezeigt. Als Referenzgas wurde ein typisches H-Erdgas verwendet (gestrichelte Linie bei der Koordinate (1.00,1.00).

Die oben stehend beschriebene Messvorrichtung zur Bestimmung physikalischer Eigenschaften und/oder brenntechnisch relevanter Grössen eines Gases und Gasgemisches ist einer neuen Kategorie zuzuteilen, nämlich "Wärmeleitfähigkeits- und Durchflussmessung mit Hilfe eines mikrothermischen Sensors, Aufsummieren der Durchflusswerte und Vergleich mit einem Volumenausfluss aus einem Referenzvolumen. Zuteilung der Erdgase zur H-Gas bzw. L-Gas Gruppe". Die verwendeten Komponenten sind kostengünstig, wodurch neue Märkte erschlossen werden können, in denen heute aus Kostengründen keine Gasqualitätssensoren eingesetzt werden. Von der Genauigkeit her sind nur wenig Einbussen gegenüber teureren, kommerziell erhältlichen Geräten zu erwarten, da hier anstatt drei noch zwei voneinander unabhängige Messwerte für die Korrelation verwendet werden.

**Patentansprüche**

1. Verfahren zur Bestimmung physikalischer Eigenschaften und/oder brenntechnisch relevanter Grössen eines Gases und Gasgemisches, wobei in dem Verfahren:

   - das Gas oder Gasgemisch aus einem Gasreservoir (4) durch eine kritische Düse (6) und über einen mikrothermischen Sensor (7) fliesst, wobei die kritische Düse und der mikrothermischen Sensor mit demselben Massenfluss beaufschlagt werden;
   - der Druckabfall im Gasreservoir (4) als Funktion der Zeit gemessen wird;
   - aus den Messwerten des Druckabfalls ein erster, von einer ersten Gruppe von physikalischen Eigenschaften des Gases oder Gasgemisches abhängiger Gaseigenschaftsfaktor ($\Gamma^*$) bestimmt wird, wobei der erste Gaseigenschaftsfaktor zum Beispiel aus einer Zeitkonstante des Druckabfalls abgeleitet wird;
   - aus dem Durchflusssignal des mikrothermischen Sensors (7) ein zweiter, von einer zweiten Gruppe von physikalischen Eigenschaften des Gases oder Gasgemisches abhängiger Gaseigenschaftsfaktor ($\Gamma$) bestimmt wird, wobei der zweite Gaseigenschaftsfaktor die Wärmekapazität ($c_p$) des Gases oder Gasgemisches enthält oder von derselben abhängig ist;
   - mit Hilfe des mikrothermischen Sensors (7) die Wärmeleitfähigkeit ($\lambda$) des Gases oder Gasgemisches bestimmt wird; und
   - aus dem ersten und/oder zweiten Gaseigenschaftsfaktor ($\Gamma^*$, $\Gamma$) und der Wärmeleitfähigkeit ($\lambda$) mittels Korrelation eine gesuchte physikalische Eigenschaft oder brenntechnisch relevante Grösse ermittelt wird.

2. Verfahren nach Anspruch 1, wobei von einem exponentiellen Abfall des gemessenen Druckes ausgegangen und der erste Gaseigenschaftsfaktor ($\Gamma^*$) aus der Zeitkonstante des Druckabfalls abgeleitet wird, und wobei der erste Gaseigenschaftsfaktor insbesondere gebildet wird, indem zusätzlich die Temperatur ($T$) gemessen und alle nicht gasabhängigen Grössen weggelassen werden.

3. Verfahren nach einem der Ansprüche 1 oder 2, wobei der zweite Gaseigenschaftsfaktor ($\Gamma$) den Quotienten Wärmekapazität ($c_p$) geteilt durch Wärmeleitfähigkeit ($\lambda$) des Gases oder Gasgemisches enthält oder von demselben abhängig ist, und wobei der zweite Gaseigenschaftsfaktor insbesondere gebildet wird, indem zusätzlich die Temperatur (T) gemessen und alle nicht gasabhängigen Grössen weggelassen werden.

4. Verfahren nach einem der Ansprüche 1 bis 3, wobei die Gaseigenschaftsfaktoren ($\Gamma^*$, $\Gamma$) durch Vergleich der Werte für das Gesamtvolumen des ausgeströmten Gases oder Gasgemisches validiert werden, indem:

- Druck und Temperatur im Gasreservoir (4) am Anfang und am Schluss der Druckabfallmessung gemessen und das ausgeflossene Normvolumen bei bekanntem Volumen des Gasreservoirs bestimmt werden;
- der mittels des mikrothermischen Sensors (7) gemessene Normdurchfluss über das Zeitintervall zwischen Anfang und Schluss der Druckabfallmessung aufsummiert wird;
- das ausgeflossene Normvolumen mit dem aufsummierten Normdurchfluss verglichen wird; und
- bei Ungleichheit entweder der erste und/oder zweite Gaseigenschaftsfaktor ($\Gamma^*$, $\Gamma$) korrigiert wird, zum Beispiel durch Korrektur des Drucksignals oder des Normvolumenflusswertes des mikrothermischen Sensors (7).

5. Verfahren zur Bestimmung physikalischer Eigenschaften und/oder brenntechnisch relevanter Grössen eines Gases oder Gasgemisches, wobei in dem Verfahren:

- das Gas oder Gasgemisch unter Druck durch eine kritische Düse (6) und über einen mikrothermischen Sensor (7) in ein Gasreservoir (4) fliesst, wobei die kritische Düse und der mikrothermische Sensor mit demselben Massenfluss beaufschlagt werden;
- die Druckzunahme im Gasreservoir (4) als Funktion der Zeit gemessen wird;
- aus den Messwerten der Druckzunahme ein erster, von einer ersten Gruppe von physikalischen Eigenschaften des Gases oder Gasgemisches abhängiger Gaseigenschaftsfaktor ($\Gamma^*$) bestimmt wird;
- aus dem Durchflusssignal des mikrothermischen Sensors (7) ein zweiter, von einer zweiten Gruppe von physikalischen Eigenschaften des Gases oder Gasgemisches abhängiger Gaseigenschaftsfaktor ($\Gamma$) bestimmt wird, wobei der zweite Gaseigenschaftsfaktor die Wärmekapazität ($c_p$) des Gases oder Gasgemisches enthält oder von derselben abhängig ist;
- mit Hilfe des mikrothermischen Sensors (7) die Wärmeleitfähigkeit ($\lambda$) des Gases oder Gasgemisches bestimmt wird; und
- aus dem ersten und/oder zweiten Gaseigenschaftsfaktor ($\Gamma^*$, $\Gamma$) und der Wärmeleitfähigkeit ($\lambda$) mittels Korrelation eine gesuchte physikalische Eigenschaft oder brenntechnisch relevante Grösse ermittelt wird.

6. Verfahren nach Anspruch 5, wobei von einer linearen Zunahme des gemessenen Druckes ausgegangen und der erste Gaseigenschaftsfaktor ($\Gamma^*$) aus einer Proportionalitätskonstante der Druckzunahme abgeleitet wird, und wobei der erste Gaseigenschaftsfaktor insbesondere gebildet wird, indem zusätzlich die Temperatur (T) und der Düsenvordruck ($p_{Nozzle}$) gemessen und alle nicht gasabhängigen Grössen weggelassen werden.

7. Verfahren nach einem der Ansprüche 5 oder 6, wobei der zweite Gaseigenschaftsfaktor ($\Gamma$) den Quotienten Wärmekapazität ($c_p$) geteilt durch Wärmeleitfähigkeit ($\lambda$) des Gases oder Gasgemisches enthält oder von demselben abhängig ist, und wobei der erste Gaseigenschaftsfaktor insbesondere gebildet wird, indem zusätzlich die Temperatur (T) gemessen und alle nicht gasabhängigen Grössen weggelassen werden.

8. Verfahren nach einem der Ansprüche 5 bis 7, wobei die Gaseigenschaftsfaktoren ($\Gamma^*$, $\Gamma$) durch Vergleich der Werte für das Gesamtvolumen des ins Gasreservoir (4) geflossenen Gases oder Gasgemisches validiert werden, indem:

- Druck und Temperatur im Gasreservoir (4) am Anfang und am Schluss der Druckzunahmemessung gemessen werden, und das ins Gasreservoir geflossene Normvolumen bei bekanntem Volumen des Gasreservoirs bestimmt wird;
- der mittels des mikrothermischen Sensors (7) gemessene Normdurchfluss über das Zeitintervall zwischen Anfang und Schluss der Druckzunahmemessung aufsummiert wird;
- das ins Gasreservoir (4) geflossene Normvolumen mit dem aufsummierten Normdurchfluss verglichen wird; und
- bei Ungleichheit entweder der erste und/oder zweite Gaseigenschaftsfaktor korrigiert wird, zum Beispiel durch Korrektur des Drucksignals oder des Normvolumenflusswertes des mikrothermischen Sensors (7).

9. Verfahren nach einem der vorangehenden Ansprüche, wobei das Verfahren zur Kalibrierung des Durchflusssignals des mikrothermischen Sensors (7) benutzt wird, indem:

- das Durchflusssignal des mikrothermischen Sensors (7) für ein bestimmtes Kalibriergas oder-Gasgemisch kalibriert wird;
- das Verhältnis ($\Gamma$ / $\Gamma^*$) des zweiten, aus dem Durchflusssignal des mikrothermischen Sensors bestimmten Gaseigenschaftsfaktors zum ersten Gaseigenschaftsfaktor für ein unbekanntes Gas oder Gasgemisch bestimmt wird; und
- die Normvoluminawerte aus der Messung der Druckabnahme oder Druckzunahme und der Messung des aufsummierten Normdurchflusses des mikrothermische Sensors (7) verglichen und dazu verwendet werden, das Verhältnis des zweiten zum ersten Gaseigenschaftsfaktor zu korrigieren und den Wert für den zweiten Gaseigenschaftsfaktor ($\Gamma$) anzupassen.

**10.** Verfahren nach einem der vorangehenden Ansprüche, wobei die gesuchte physikalische Eigenschaft die Dichte oder die Wärmeleitfähigkeit oder die Wärmekapazität oder die Viskosität des Gases oder Gasgemisches ist, und/oder wobei die brenntechnisch relevante Grösse der Energieinhalt oder der Brennwert oder der Wobbe-Index oder die Methanzahl oder der Luftbedarf des Gases oder Gasgemisches ist.

**11.** Verfahren nach einem der vorangehenden Ansprüche, wobei die gesuchte physikalische Eigenschaft oder brenntechnisch relevante Grösse (Q) mittels einer Korrelationsfunktion $Q = f_{corr}\,(\Gamma, \Gamma^*, \lambda) = const \cdot \Gamma^r \cdot \Gamma^{*s} \cdot \lambda^t$ ermittelt wird, wobei $r$, s und $t$ Exponenten sind und *const* eine Konstante ist.

**12.** Verfahren nach einem der vorangehenden Ansprüche, wobei der Druck im Gasreservoir (4) zu Beginn der Druckabfallmessung grösser ist als der kritische Druck ($p_{krit}$) der kritischen Düse (6) und der Aussendruck nach der kritischen Düse kleiner als die Hälfte des kritischen Druckes, oder wobei der Druck im Gasreservoir (4) zu Beginn der Druckzunahmemessung kleiner ist als die Hälfte des kritischen Druckes ($p_{krit}$) der kritischen Düse (6) und der Druck vor der kritischen Düse grösser ist als der kritische Druck.

**13.** Messvorrichtung zur Bestimmung physikalischer Eigenschaften und/oder brenntechnisch relevanter Grössen eines Gases oder Gasgemisches mit einer Auswerteeinheit (11), die zur Ausführung eines Verfahrens gemäss einem der Ansprüche 1 bis 12 eingerichtet ist, und mit einem Gasreservoir (4), das mit einem Drucksensor (8) versehen ist, einer kritischen Düse (6) und einem mikrothermischen Sensor (7) zur Messung des Durchflusses und der Wärmeleitfähigkeit, wobei das Gasreservoir für die Messung mit der kritischen Düse und dem mikrothermischen Sensor verbunden ist.

## Claims

**1.** Method for determining physical properties and/or quantities relevant to combustion of gas and gas mixtures, whereby in the method:

- the gas or gas mixture flows from a gas reservoir (4) through a critical nozzle (6) and past a microthermal sensor, with the same mass flow being applied to the critical nozzle and the microthermal sensor;
- the pressure drop in the gas reservoir (4) is measured as a function of time;
- a first gas property factor ($\Gamma^*$), dependent on a first group of physical properties of the gas or gas mixture, is determined on the basis of the measured values of the pressure drop, with the first gas property factor being derived, for example, from a time constant of the pressure drop;
- a second gas property factor ($\Gamma$), dependent on a second group of physical properties of the gas or gas mixture, is determined from the flow signal of the microthermal sensor, with the second gas property factor containing the heat capacity ($c_p$) of the gas or gas mixture, or being dependent on the same;
- the thermal conductivity ($\lambda$) of the gas or gas mixture is determined with the aid of the microthermal sensor (7); and
- a desired physical property or quantity relevant to combustion is determined from the first and/or second gas property factor ($\Gamma^*$, $\Gamma$) and thermal conductivity ($\lambda$) through correlation.

**2.** Method according to claim 1, in which the starting point is an exponential decline of the measured pressure and the first gas property factor ($\Gamma^*$) is derived from the time constant of the pressure drop, in which case the first gas property factor is formed in particular by measuring the temperature (T) and by omitting all gas-unrelated variables.

3. Method according to one of the claims 1 or claim 2, in which the second gas property factor ($\Gamma$) contains the quotient of heat capacity ($c_p$) divided by thermal conductivity ($\lambda$) of the gas or gas mixture, or is dependent on the same, and in which the second gas property factor is formed by measuring the temperature ($T$) additionally and by omitting all gas-unrelated variables.

4. Method according to one of the claims 1 to 3, wherein the gas property factors ($\Gamma^*$, $\Gamma$) are validated by comparing the values for the total volume of released gas or gas mixture by:

   - measuring the pressure and temperature in the gas reservoir (4) at the start and end of the pressure drop reading and by determining the released standard volume by reference to the known volume of the gas reservoir;
   - summing up the standard flow measured with the microthermal sensor (7) during the time interval between the start and end of the pressure drop reading;
   - comparing the released standard volume to the summed up standard volume; and
   - in case of a discrepancy, by adjusting the first and/or the second gas property factor ($\Gamma^*$, $\Gamma$) for example, by adjusting the pressure signal or the standard flow variable of the microthermal sensor (7).

5. Method for determining physical properties and/or quantities relevant to combustion of gas or gas mixtures, whereby in the method:

   - the gas or gas mixture flows under pressure through a critical nozzle (6) and past a microthermal sensor (7) into a gas reservoir (4), with the same mass flow being applied to the critical nozzle and the microthermal sensor;
   - the pressure increase in the gas reservoir (4) is measured as a function of time;
   - a first gas property factor ($\Gamma^*$), dependent on a first group of physical properties of the gas or gas mixture, is determined by reference to the measured values of the pressure increase;
   - a second gas property factor ($\Gamma$), dependent on a second group of physical properties of the gas or gas mixture, is determined from the flow signal of the microthermal sensor, with the second gas property factor containing the heat capacity ($c_p$) of the gas or gas mixture, or being dependent on the same;
   - the thermal conductivity ($\lambda$) of the gas or gas mixture is determined with the aid of the microthermal sensor (7); and
   - a desired physical property or quantity relevant to combustion is determined from the first and/or second gas property factor ($\Gamma^*$, $\Gamma$) and thermal conductivity ($\lambda$) through correlation.

6. Method according to claim 5, where the starting point is a linear increase of the measured pressure and the first gas property factor ($\Gamma^*$) is derived from a proportionality constant of the pressure increase, and in which case the first gas property factor is formed by measuring, for example, in addition the temperature *(T)* and the nozzle inlet pressure ($p_{Nozzle}$), and by omitting all gas-unrelated variables.

7. Method according to one of the claims 5 or claim 6, in which the second gas property factor ($\Gamma$) contains the quotient of heat capacity ($c_p$) divided by thermal conductivity ($\lambda$) of the gas or gas mixture, or is dependent on the same, and in which the second gas property factor is formed in particular by additionally measuring the temperature (T) and by omitting all gas-unrelated variables.

8. Method according to one of the claims 5 to 7, where the gas property factors ($\Gamma^*$, $\Gamma$) are validated by comparing the values for the total volume of the gas or gas mixture fed into the gas reservoir (4) by:

   - measuring the pressure and temperature in the gas reservoir (4) at the start and end of the pressure increase reading and by determining the released standard volume fed into the gas reservoir by reference to the known volume of the gas reservoir;
   - summing up the standard flow measured with the microthermal sensor (7) during the time interval between the start and end of the pressure increase reading;
   - comparing the standard volume fed into the gas reservoir (4) to the summed up standard volume; and
   - in the case of a discrepancy, by adjusting the first and/or the second gas property factor, for example, by adjusting the pressure signal or the standard flow variable of the microthermal sensor (7).

9. Method according to one of the preceding claims, wherein the method is used for calibrating the flow signal of the microthermal sensor (7), by:

   - calibrating the flow signal of the microthermal sensor (7) for a specific calibration gas or gas mixture;

- determining the ratio ($\Gamma$ / $\Gamma^*$) of the second gas property factor, determined on the basis of the flow signal of the microthermal sensor, to the first gas property factor for an unknown gas or gas mixture; and
- comparing the standard volume values from the reading of the pressure drop or pressure increase and the reading of the summed up standard flow of the microthermal sensor (7), which are then used to adjust the ratio of the second gas property factor to the first, and to adapt the value for the second gas property factor ($\Gamma$).

10. Method according to one of the preceding claims where the desired physical property is the density or the thermal conductivity or the heat capacity or the viscosity of the gas or gas mixture, and/or where the quantity relevant to combustion is the energy content or the calorific value or the Wobbe index or the methane number or the air requirement of the gas or gas mixture.

11. Method according to one of the preceding claims, where the desired physical property or the quantity relevant to combustion ($Q$) is determined by aid of a correlation function $Q = f_{corr}$ ($\Gamma$, $\Gamma^*$, $\lambda$) = $const \cdot \Gamma^r \cdot \Gamma^{*s} \cdot \lambda^t$, wherein $r, s$ and $t$ are exponents, and *const* is a constant.

12. Method according to one of the preceding claims, where the pressure in the gas reservoir (4) is higher at the start of the pressure drop reading than the critical pressure ($p_{crit}$) of the critical nozzle (6) and the external pressure downstream of the critical nozzle is less than half of the critical pressure, or where the pressure in the gas reservoir (4) at the start of the pressure increase reading is lower than the critical pressure ($p_{crit}$) of the critical nozzle (6) and the pressure upstream of the critical nozzle is higher than the critical pressure.

13. Measuring apparatus for determining physical properties and/or quantities relevant to combustion of gas or gas mixtures comprising an analyzer unit (11) that is configured to carry out a procedure in accordance with one of the claims 1 to 12 and a gas reservoir (4) that is equipped with a pressure sensor (8), a critical nozzle (6) and a microthermal sensor (7) to measure the flow and thermal conductivity, in which case the gas reservoir is connected to the critical nozzle and the microthermal sensor for the purposes of the measurement.

## Revendications

1. Procédé pour la détermination de caractéristiques physiques et/ou de grandeurs pertinentes pour la combustion d'un gaz ou mélange gazeux, procédé dans lequel :

   - le gaz ou mélange gazeux s'écoule à partir d'un réservoir de gaz (4) à travers une buse (6) critique et en passant par un capteur micro-thermique (7), la buse critique et le capteur micro-thermique étant alimentés avec le même flux massique ;
   - la chute de pression dans le réservoir de gaz (4) est mesurée en fonction du temps ;
   - à partir des valeurs de mesure de la chute de pression, un premier facteur de caractéristique de gaz ($\Gamma^*$) dépendant d'un premier groupe de caractéristiques physiques du gaz ou mélange gazeux est déterminé, le premier facteur de caractéristique de gaz étant par exemple dérivé d'une constante de temps de la chute de pression ;
   - à partir du signal de débit du capteur micro-thermique (7), un deuxième facteur de caractéristique de gaz (r) dépendant d'un deuxième groupe de caractéristiques physiques du gaz ou mélange gazeux est déterminé, le deuxième facteur de caractéristique de gaz contenant la capacité thermique ($c_p$) du gaz ou mélange gazeux ou étant dépendant de celle-ci ;
   - la conductibilité thermique ($\lambda$) du gaz ou mélange gazeux est déterminée à l'aide du capteur micro-thermique (7) ; et
   - à partir du premier et/ou deuxième facteur de caractéristique de gaz ($\Gamma^*$, $\Gamma$) et de la conductibilité thermique ($\lambda$), une caractéristique physique recherchée ou grandeur pertinente pour la combustion recherchée est déterminée au moyen d'une corrélation.

2. Procédé selon la revendication 1, dans lequel on part d'une chute exponentielle de la pression mesurée, et le premier facteur de caractéristique de gaz ($\Gamma^*$) étant dérivé de la constante de temps de la chute de pression, et le premier facteur de caractéristique de gaz étant en particulier formé en mesurant en plus la température ($T$) et en négligeant toutes les grandeurs ne dépendant pas du gaz.

3. Procédé selon l'une des revendications 1 ou 2, le deuxième facteur de caractéristique de gaz (r) contenant le quotient de la capacité thermique ($c_p$) divisée par la conductibilité thermique ($\lambda$) du gaz ou mélange gazeux ou étant dépendant de celui-ci, et le deuxième facteur de caractéristique de gaz étant en particulier formé en mesurant en plus la température (*T*) et en négligeant toutes les grandeurs ne dépendant pas du gaz.

4. Procédé selon l'une des revendications 1 à 3, les facteurs de caractéristique de gaz ($\Gamma^*$, $\Gamma$) étant validés par la comparaison des valeurs du volume total du gaz ou mélange gazeux évacué

   - en mesurant la pression et la température dans le réservoir de gaz (4) au début et à la fin de la mesure de la chute de pression et en déterminant le volume normalisé écoulé pour un volume connu du réservoir de gaz ;
   - en totalisant le débit normalisé mesuré au moyen du capteur micro-thermique (7) sur l'intervalle de temps entre le début et la fin de la mesure de la chute de pression ;
   - en comparant le volume normalisé écoulé au débit normalisé totalisé ; et,
   - en cas d'inégalité, en corrigeant soit le premier soit le deuxième facteur de caractéristique de gaz ($\Gamma^*$, $\Gamma$) ou les deux, par exemple par la correction du signal de pression ou de la valeur de flux volumique normalisé du capteur micro-thermique (7).

5. Procédé pour la détermination de caractéristiques physiques et/ou de grandeurs pertinentes pour la combustion d'un gaz ou mélange gazeux, procédé dans lequel :

   - le gaz ou mélange gazeux s'écoule sous pression vers un réservoir de gaz (4) à travers une buse (6) critique et en passant par un capteur micro-thermique (7), la buse critique et le capteur micro-thermique étant alimentés avec le même flux massique ;
   - l'augmentation de pression dans le réservoir de gaz (4) est mesurée en fonction du temps ;
   - à partir des valeurs de mesure de l'augmentation de pression, un premier facteur de caractéristique de gaz ($\Gamma^*$) dépendant d'un premier groupe de caractéristiques physiques du gaz ou mélange gazeux est déterminé ;
   - à partir du signal de débit du capteur micro-thermique (7), un deuxième facteur de caractéristique de gaz (r) dépendant d'un deuxième groupe de caractéristiques physiques du gaz ou mélange gazeux est déterminé, le deuxième facteur de caractéristique de gaz contenant la capacité thermique ($c_p$) du gaz ou mélange gazeux ou étant dépendant de celle-ci ;
   - la conductibilité thermique ($\lambda$) du gaz ou mélange gazeux est déterminée à l'aide du capteur micro-thermique (7) ; et
   - à partir du premier et/ou deuxième facteur de caractéristique de gaz ($\Gamma^*$, $\Gamma$) et de la conductibilité thermique ($\lambda$), une caractéristique physique recherchée ou grandeur pertinente pour la combustion recherchée est déterminée au moyen d'une corrélation.

6. Procédé selon la revendication 5, dans lequel on part d'une augmentation linéaire de la pression mesurée, et le premier facteur de caractéristique de gaz ($\Gamma^*$) est dérivé d'une constante de proportionnalité de l'augmentation de pression, et le premier facteur de caractéristique de gaz étant en particulier formé en mesurant en plus la température (T) et la pression en amont de la buse ($p_{nozzle}$) et en négligeant toutes les grandeurs ne dépendant pas du gaz.

7. Procédé selon l'une des revendications 5 ou 6, le deuxième facteur de caractéristique de gaz (r) contenant le quotient de la capacité thermique ($c_p$) divisée par la conductibilité thermique ($\lambda$) du gaz ou mélange gazeux ou étant dépendant de celui-ci, et le premier facteur de caractéristique de gaz étant en particulier formé en mesurant en plus la température (T) et en négligeant toutes les grandeurs ne dépendant pas du gaz.

8. Procédé selon l'une des revendications 5 à 7, les facteurs de caractéristique de gaz ($\Gamma^*$, $\Gamma$) étant validés par la comparaison des valeurs du volume total du gaz ou mélange gazeux s'écoulant dans le réservoir de gaz (4)

   - en mesurant la pression et la température dans le réservoir de gaz (4) au début et à la fin de la mesure de l'augmentation de pression, et en déterminant le volume normalisé écoulé dans le réservoir de gaz pour un volume connu du réservoir de gaz ;
   - en totalisant le débit normalisé mesuré au moyen du capteur micro-thermique (7) sur l'intervalle de temps entre le début et la fin de la mesure de l'augmentation de pression ;
   - en comparant le volume normalisé écoulé dans le réservoir de gaz (4) au débit normalisé totalisé ; et,
   - en cas d'inégalité, en corrigeant soit le premier soit le deuxième facteur de caractéristique de gaz ($\Gamma^*$, $\Gamma$) ou les deux, par exemple par la correction du signal de pression ou de la valeur de flux volumique normalisé du capteur micro-thermique (7).

9. Procédé selon l'une des revendications précédentes, le procédé étant utilisé pour l'étalonnage du signal de débit du capteur micro-thermique (7)

- en étalonnant le signal de débit du capteur micro-thermique (7) pour un gaz ou mélange gazeux d'étalonnage défini ;
- en déterminant le rapport (Γ / Γ*) du deuxième facteur de caractéristique de gaz déterminé à partir du signal de débit du capteur micro-thermique au premier facteur de caractéristique de gaz pour un gaz ou mélange gazeux inconnu ; et
- en comparant les valeurs de volume normalisé issues de la mesure de la diminution de pression ou de l'augmentation de pression et de la mesure du débit normalisé totalisé du capteur micro-thermique (7) et en les utilisant pour corriger le rapport du premier au deuxième facteur de caractéristique de gaz et pour adapter la valeur du deuxième facteur de caractéristique de gaz (r).

10. Procédé selon l'une des revendications précédentes, la caractéristique physique recherchée étant la densité ou la conductibilité thermique ou la capacité thermique ou la viscosité du gaz ou mélange gazeux, et/ou la grandeur pertinente pour la combustion étant la teneur énergétique ou le pouvoir calorifique ou l'indice de Wobbe ou l'indice de méthane ou la demande en air du gaz ou mélange gazeux.

11. Procédé selon l'une des revendications précédentes, la caractéristique physique recherchée ou la grandeur ($Q$) pertinente pour la combustion étant déterminée au moyen d'une fonction de corrélation $Q = f_{corr}(\Gamma,\Gamma^*,\lambda) = const\cdot\Gamma^r\cdot\Gamma^{*s}\cdot\lambda^t$, r, s et t étant des exposants et *const* étant une constante.

12. Procédé selon l'une des revendications précédentes, la pression dans le réservoir de gaz (4) étant au début de la mesure de la chute de pression supérieure à la pression critique ($p_{krit}$) de la buse (6) critique, et la pression extérieure en aval de la buse critique étant inférieure à la moitié de la pression critique, ou la pression dans le réservoir de gaz (4) étant au début de la mesure de l'augmentation de pression inférieure à la moitié de la pression critique ($p_{krit}$) de la buse (6) critique, et la pression en amont de la buse critique étant supérieure à la pression critique.

13. Dispositif de mesure pour la détermination de caractéristiques physiques et/ou de grandeurs pertinentes pour la combustion d'un gaz ou mélange gazeux avec une unité d'analyse (11) qui est aménagée pour la réalisation d'un procédé selon l'une des revendications 1 à 12, et avec un réservoir de gaz (4) qui est muni d'un capteur de pression (8), une buse (6) critique et un capteur micro-thermique (7) pour la mesure du débit et de la conductibilité thermique, le réservoir de gaz étant, pour la mesure, raccordé à la buse critique et au capteur micro-thermique.

**Fig. 1a**

**Fig. 1b**

**Fig. 2**

**Fig. 3**

**Fig. 4**

**Fig. 5a**

**Fig. 5b**

**Fig. 6**

**Fig. 7**

**Fig. 8a**

**Fig. 8b**

**Fig. 9**

**Fig. 10**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2015056 A1

- WO 2004036209 A1

**In der Beschreibung aufgeführte Nicht-Patentliteratur**

- **U. WERNEKINCK.** Gasmessung und Gasabrechung. Vulkan Verlag, 2009
- **D. MATTER ; B. KRAMER ; T. KLEINER ; B. SABBATTINI ; T. SUTER.** Mikroelektronischer Haushaltsgaszähler mit neuer Technologie. *Technisches Messen,* 2004, vol. 71 (3), 137-146

- **KERSON HUANG.** Statistical Mechanics. John Wiley & Sons, 1987